# EUROPEAN PATENT SPECIFICATION

(11) **EP 3 720 453 B1**
(45) Date of publication and mention of the grant of the patent: **10.07.2024**
(21) Application number: 18836732.0
(22) Date of filing: 04.12.2018
(51) Int. Cl.: C07K 14/715, C12N 5/0783, C12N 15/88, C12N 15/11, C07K 16/28, A61K 39/00

(54) **MODIFIED LYMPHOCYTES**
MODIFIZIERTE LYMPHOZYTEN
LYMPHOCYTES MODIFIÉS

(30) Priority: 05.12.2017 US 201762594993 P
(43) Date of publication of application: 14.10.2020
(73) Proprietor: Caribou Biosciences, Inc., Berkeley, CA 94710 (US)
(72) Inventor: STENGEL, Katharina, Berkeley CA 94710 (US)
(74) Representative: Grund, Martin
(86) International application number: PCT/US2018/063941
(87) International publication number: WO 2019/113132

(56) References cited:
- WO-A1-2017/064222
- US-A1- 2014 356 956
- SHEN CHI ET AL: "A CRISPR-Based Toolbox for Studying T Cell Signal Transduction", BIOMED RESEARCH INTERNATIONAL, vol. 2016, 2016, pages 1 - 10, XP055570357, ISSN: 2314-6133, DOI: 10.1155/2016/5052369
- B. LEE ET AL: "Quantification of CD4, CCR5, and CXCR4 levels on lymphocyte subsets, dendritic cells, and differentially conditioned monocyte-derived macrophages", PROCEEDINGS OF THE NATIONAL ACADEMY OF SCIENCES OF THE UNITED STATES OF AMERICA, vol. 96, no. 9, 27 April 1999 (1999-04-27), US, pages 5215 - 5220, XP055570624, ISSN: 0027-8424, DOI: 10.1073/pnas.96.9.5215
- E. K. MOON ET AL: "Expression of a Functional CCR2 Receptor Enhances Tumor Localization and Tumor Eradication by Retargeted Human T cells Expressing a Mesothelin-Specific Chimeric Antibody Receptor", CLINICAL CANCER RESEARCH, vol. 17, no. 14, 15 July 2011 (2011-07-15), pages 4719 - 4730, XP055067031, ISSN: 1078-0432, DOI: 10.1158/1078-0432.CCR-11-0351
- IMRAN SIDDIQUI ET AL: "Enhanced recruitment of genetically modified CX3CR1-positive human T cells into Fractalkine/CX3CL1 expressing tumors: importance of the chemokine gradient", JOURNAL FOR IMMUNOTHERAPY OF CANCER, vol. 4, no. 1, 19 April 2016 (2016-04-19), XP055535227, DOI: 10.1186/s40425-016-0125-1
- MARIE F. LA RUSSA ET AL: "The New State of the Art: Cas9 for Gene Activation and Repression", MOLECULAR AND CELLULAR BIOLOGY., vol. 35, no. 22, 15 November 2015 (2015-11-15), US, pages 3800 - 3809, XP055570755, ISSN: 0270-7306, DOI: 10.1128/MCB.00512-15
- ALBERT LO ET AL: "Genetic and epigenetic control of gene expression by CRISPR-Cas systems", F1000RESEARCH, vol. 6, 25 May 2017 (2017-05-25), pages 747, XP055570757, DOI: 10.12688/f1000research.11113.1

## Description

### TECHNICAL FIELD

The present invention relates to modified human T cells comprising at least one ectopically expressed endogenous chemokine receptor not expressed in an unmodified human T cell, compositions comprising such modified human T cells and their use in the treatment of cancer.

### BACKGROUND

Lymphocytes are types of leukocytes (white blood cells) that function in the vertebrate immune system. Lymphocytes include T cells for cell-mediated, cytotoxic adaptive immunity; natural killer (NK) cells that function in cell-mediated, cytotoxic innate immunity; and B cells, for humoral, antibody-driven adaptive immunity. Receptors expressed on the surface of lymphocytes bind to their specified ligands and, upon binding, lymphocytes propagate to produce additional lymphocytes or further differentiate into additional immune system cells. Additionally, receptor ligand binding directs cytotoxic lymphocytes such as T cells and NK cells, to kill target cells.

Adoptive cell transfer (ACT) is a rapidly emerging immunotherapy approach that typically uses a patient's immune cells to treat cancer. Such methods utilize, for example, chimeric antigen receptor T cells (CAR-T cells), tumor infiltrating lymphocytes (TILs), T cell receptor-engineered T cells (TCRs), or engineered NK cells. ACT has been shown to be effective for some liquid tumors. However, the efficacy of ACT to treat solid tumors has proven more challenging. Currently, efficient trafficking or "homing in" of cytotoxic T cells to the tumor or tumor microenvironment is a hurdle.

In general, lymphocytes use cell surface chemokine receptors to sense cognate chemokines secreted by cells for directed homing on their target sites. Although the number of chemokines and cognate chemokine receptors is large, each lymphocyte type expresses only a limited set of chemokine receptors on their surface, despite the fact that the genes encoding these receptors are present in the lymphocyte genome.

For example, particular chemokines are expressed and secreted in abundance by some cancer cells or by the tumor microenvironment. However, lymphocytes, such as cytotoxic T cells, often do not express a matched/cognate cell surface chemokine receptor and hence cannot sense chemokines expressed by the tumor. In this case, trafficking of the cytotoxic lymphocytes to the tumor sites and tumor microenvironment is inefficient and negatively impacts treatment efficacy.

First attempts have been made to modify lymphocytes to promote expression of chemokine receptor genes that allow and improve tumor targeting of such lymphocytes. However, these attempts have so far been restricted to exogenous genes introduced into lymphocytes (Moon et al., 2011).

Accordingly, there is a need for lymphocytes that are modified to express cell surface endogenous genes normally silent or transcriptionally repressed, in order to target lymphocytes to a particular tumor or tumor microenvironment.

### SUMMARY

The present invention pertains to human T cells that have been modified in order to provide more efficacious immunotherapeutic treatments for cell proliferative diseases, such as cancer. Accordingly, the invention provides a modified human T cell comprising at least one ectopically expressed endogenous chemokine receptor not expressed in an unmodified human T cell, wherein the modified human T cell is produced by a method comprising introducing into a human T cell a polynucleotide encoding a chimeric antigen receptor (CAR); and an artificial transcription factor (ATF) complex comprising a catalytically inactive CRISPR-associated protein (dCas), a nucleic acid-targeting nucleic acid (NATNA), and an effector domain; whereby the ATF complex binds to a nucleic acid proximal to a transcriptional start site (TSS) of an endogenous chemokine receptor gene, wherein the at least one ectopically expressed endogenous chemokine receptor is selected from the group consisting of CCR1, CCR2, CCR3, CCR4, CCR5, CXCR4 and DARC.

In further embodiments, a modified human T cell is provided, wherein the isolated human T cell comprises a modification whereby an endogenous CCR2-encoding gene in the T cell genome is expressed, wherein the CCR2-encoding gene is not expressed in a naturally occurring T cell, and further wherein the modification does not comprise an engineered insertion of the CCR2-encoding gene into the T cell genome.

In further embodiments, a method of preparing a modified human T cell as described in the embodiments above, is provided. The method comprises: introducing into the T cell a polynucleotide encoding a chimeric antigen receptor (CAR); and an artificial transcription factor (ATF) complex comprising a catalytically inactive CRISPR-associated protein (dCas), a nucleic acid-targeting nucleic acid (NATNA), and an effector domain, wherein the ATF complex binds to a nucleic acid proximal to a transcriptional start site (TSS) of an endogenous chemokine receptor gene in the genome of the T cell to promote expression of the gene; and selecting for T cells that express the selected endogenous chemokine receptor on the lymphocyte cell surface.

In certain embodiments, the effector domain is from VP16 or VP64. In other embodiments, the effector domain comprises a MS2-binding RNA.

In yet further embodiments of the methods, the selected endogenous chemokine receptor-encoding gene encodes CCR1, CCR2, CCR3, CCR4, CCR5, CXCR4, or DARC.

In additional embodiments, a method of preparing a modified human T cell as described above is provided. The method comprises: introducing into the T cell a polynucleotide encoding a chimeric antigen receptor (CAR); and an artificial transcription factor (ATF) complex comprising a dCas9/sgRNA complex and a VP64 effector domain, wherein the complex binds to a nucleic acid sequence proximal to a transcriptional start site (TSS) of a selected endogenous CCR2-encoding gene in the genome of the T cell to promote expression of the gene; and selecting for T cells that express the CCR2 receptor on the T cell surface.

In additional embodiments, a modified human T cellis provided as produced by any of the methods above.

In yet further embodiments, a composition is provided. The composition comprises a modified human T cell as described above; and a pharmaceutically acceptable excipient.

In further embodiments, a composition comprising a modified human T cell as described above and a pharmaceutically acceptable excipient for use in the treatment of cancer is provided. comprising administering a therapeutically effective amount of said composition as described above, to a human subject. In some embodiments, the composition is administered parenterally, such as intravenously.

In certain embodiments, the cancer is bone cancer, breast cancer, colorectal cancer, gastric cancer, liver cancer, lung cancer, ovarian cancer, pancreatic cancer, prostate cancer, skin cancer, testicular cancer, and vaginal cancer.

These aspects and other embodiments of the invention will readily occur to those of ordinary skill in the art in view of the disclosure herein.

### DETAILED DESCRIPTION

The invention is defined by the appended claims.

It is to be understood that the terminology used herein is for the purpose of describing particular embodiments only, and is not intended to be limiting. As used in the present Specification and the appended claims, the singular forms "a," "an" and "the" include plural referents unless the context clearly dictates otherwise. Thus, for example, reference to "a lymphocyte" includes one or more lymphocytes, and the like.

Unless defined otherwise, all technical and scientific terms used herein have the same meaning as commonly understood by one of ordinary skill in the art to which the invention pertains. Although other methods and materials similar, or equivalent, to those described herein can be used in the practice of the present invention, preferred materials and methods are described herein.

In view of the teachings of the present Specification, one of ordinary skill in the art can apply conventional techniques of immunology, biochemistry, chemistry, molecular biology, microbiology, cell biology, genomics, and recombinant polynucleotides, as taught, for example, by the following standard texts: Antibodies: A Laboratory Manual, Second edition, E. A. Greenfield, 2014, Cold Spring Harbor Laboratory Press, ISBN 978-1-936113-81-1; Culture of Animal Cells: A Manual of Basic Technique and Specialized Applications, 6th Edition, R. I. Freshney, 2010, Wiley-Blackwell, ISBN 978-0-470-52812-9; Transgenic Animal Technology, Third Edition: A Laboratory Handbook, 2014, C. A. Pinkert, Elsevier, ISBN 978-0124104907; The Laboratory Mouse, Second Edition, 2012, H. Hedrich, Academic Press, ISBN 978-0123820082; Manipulating the Mouse Embryo: A Laboratory Manual, 2013, R. Behringer, et al., Cold Spring Harbor Laboratory Press, ISBN 978-1936113019; PCR 2: A Practical Approach, 1995, M. J. McPherson, et al., IRL Press, ISBN 978-0199634248; Methods in Molecular Biology (Series), J.M. Walker, ISSN 1064-3745, Humana Press; RNA: A Laboratory Manual, 2010, D. C. Rio , et al., Cold Spring Harbor Laboratory Press, ISBN 978-0879698911; Methods in Enzymology (Series), Academic Press; Molecular Cloning: A Laboratory Manual (Fourth Edition), 2012, M. R. Green, et al., Cold Spring Harbor Laboratory Press, ISBN 978-1605500560; Bioconjugate Techniques, Third Edition, 2013, G. T. Hermanson, Academic Press, ISBN 978-0123822390; Methods in Plant Biochemistry and Molecular Biology, 1997, W. V. Dashek, CRC Press, ISBN 978-0849394805.

By "lymphocyte" is meant a leukocyte (white blood cell) that is part of the vertebrate immune system. Also encompassed by the term "lymphocyte" is a hematopoietic stem cell that gives rise to lymphoid cells. Lymphocytes include T cells for cell-mediated, cytotoxic adaptive immunity, such as CD4+ and/or CD8+ cytotoxic T cells; alpha/beta T cells and gamma/delta T cells; regulatory T cells, such as Treg cells; natural killer (NK) cells that function in cell-mediated, cytotoxic innate immunity; and B cells, for humoral, antibody-driven adaptive immunity. The lymphocyte can be a mammalian cell, such as a human cell. The term "lymphocyte" also encompasses genetically modified T cells, modified to produce chimeric antigen receptors (CARs) on the T cell surface (CAR-T cells). These CAR-T cells recognize specific antigens on a target cell surface, such as a tumor cell surface. The CAR comprises an extracellular ligand binding domain, a hinge region, a transmembrane region and an intracellular signaling region. The extracellular ligand binding domain typically comprises a single-chain immunoglobulin variable fragment(s) (scFv) or other ligand binding domain. The hinge region generally comprises a polypeptide hinge of variable length such as one or more amino acids, a CD8alpha or an IgG4 region (or others) and combinations thereof. The transmembrane domain typically contains a transmembrane region derived from CD8 alpha, CD28, or other transmembrane proteins and combinations thereof. The intracellular signaling domain consists of one or more intracellular signaling domains such as CD28, 4-1BB, CD3 zeta, OX40, or other intracellular signaling domains, and combinations thereof. When the extracellular ligand binding domain binds to a cognate ligand, the intracellular signaling domain of the CAR activates the lymphocyte.

Also encompassed by the term "lymphocyte," as used herein, are T cell receptor engineered T cells (TCRs), genetically engineered to express a specific, naturally occurring or engineered T-cell receptor that can recognize protein or (glyco)lipid antigens of target cells. Small pieces of these antigens, such as peptides or fatty acids, are shuttled to the target cell surface and presented to the T cell receptors as part of the MHC complex. T cell receptor binding to antigen-loaded MHC complexes activates the lymphocyte.

Tumor infiltrating lymphocytes (TILs) are also encompassed by the term "lymphocyte," as used herein. TILs are immune cells that have penetrated the environment in and around a tumor ("the tumor microenvironment"). TILs are typically isolated from tumor cells and the tumor microenvironment and are selected *in vitro* for high reactivity against tumor antigens. TILs are grown *in vitro* under conditions that overcome the tolerizing influences that exist *in vivo* and are then introduced into a subject for treatment.

CARs can also be incorporated into TILs, NK cells or TCRs resulting in CAR TILs, CAR- NK cells, or TCR engineered CAR-T cells.

Lymphocyte activation occurs when lymphocytes are triggered through antigen-specific receptors on their cell surface. This causes the cells to proliferate and differentiate into specialized effector lymphocytes. Such "activated" lymphocytes are typically characterized by a set of receptors on the surface of the lymphocyte. Surface markers for activated T cells include CD3, CD4, CD8, PD1, IL2R, and others. Activated cytotoxic lymphocytes can kill target cells after binding cognate receptors on the surface of target cells.

By an "endogenous chemokine receptor-encoding gene" is meant a gene that is naturally present in the lymphocyte genome, wherein the gene codes for a receptor that, when expressed and present on the lymphocyte cell surface, is specific for and targets the lymphocyte to a particular chemokine that is found on the cell surface or is secreted by a tumor cell or by cells in the tumor microenvironment. Many such chemokine receptor genes are known and, depending on the type of lymphocyte, may or may not be expressed in the naturally occurring lymphocyte *in vivo* even though present in the lymphocyte genome. Such endogenous genes that are present but not expressed are also called "silent" genes herein wherein expression is typically suppressed by a transcriptional repressor. By "not expressed" is meant that expression of the gene product in question is not detectable, using for example a FACS assay performed *in vitro* on an isolated lymphocyte, such as an assay as described in Example 4 herein.

By "insertion" of a cell surface receptor-encoding gene into the lymphocyte cell genome is meant that a lymphocyte is modified to express the gene by inserting an expression cassette into the lymphocyte genome that expresses the endogenous gene, e.g., using genome editing techniques such as recombinant DNA technology and CRISPR-Cas gene editing. Thus, the modification of the present invention does not comprise an engineered insertion of the endogenous chemokine receptor-encoding gene into the lymphocyte cell genome in order to express the particular chemokine receptor, but rather is accomplished by targeting the endogenous transcription machinery of the cell to cause ectopic expression of the chemokine receptor encoded by the endogenous chemokine receptor-encoding gene. "Ectopic expression" means abnormal gene expression in a cell type, tissue type, or developmental stage in which the gene is not usually expressed.

By "artificial transcriptional activator (ATA)" or an "artificial transcription factor (ATF)," as used herein, is meant a complex capable of recruiting RNA polymerase II holoenzyme to genes with which they are associated thereby causing ectopic expression of the gene of interest. Such activators include at least two components: (1) a catalytically inactive polynucleotide binding domain that either directly recognizes cognate nucleotide sequences and can bind to these sequences, or a polynucleotide binding domain that is guided to such sequences for binding (e.g., a nucleoprotein complex comprising a nucleic acid binding domain and a NATNA as defined herein); and (2) an activation domain (also termed "effector domain") that interacts with a variety of proteins that constitute the transcriptional machinery to upregulate transcription.

By "catalytically inactive polynucleotide binding domain" is meant a molecule that binds to, but does not cleave, the nucleic acid target site bound by the binding domain. Representative examples of such domains are detailed herein.

ATFs using CRISPR systems have been designed e.g., by fusing a CRISPR transcription activation factor to a complex including a single-guide RNA and a catalytically inactive CRISPR-associated protein. See, *e.g.,* Mali et al. Nature Biotechnology (2013) 31:833-838. Other ATFs have been designed by replacing naturally occurring, endogenous DNA binding domains (DBDs) with protein DBDs (Beerli et al., Nat. Biotechnol. (2002) 20:135-141); with synthetic variants such as peptide nucleic acids (Liu et al., Chem. Biol. (2003) 10:909-916); with triplex-forming oligonucleotides (Kuznetsova et al., Nucleic Acids Res. (1999) 27:3995-4000; Stanojevic et al., Biochemistry (2002) 41:7209-7216); and with hairpin polyamides (Mapp et al., Proc. Natl. Acad. Sci. USA (2000) 97:3930-3935). In addition, artificial activators that function only in the presence of a small molecule have been developed and offer some control over the timing of gene activation, thus serving as a substitute for the signaling pathways that regulate natural activation domain function (Lin et al., J. Am. Chem. Soc. (2003) 125:612-613). Non- limiting examples of activators include CRISPR-associated (Cas) protein transcription factors such as in CRISPRa and CASCADEa; zinc finger transcription factors; such as used in ZnFa; TALE transcription factors, such as used in TALEa; meganuclease transcription factors; and various small molecules, antibodies, polypeptides or oligonucleotides, as described herein.

As used herein, the terms "cytokine" and "chemokine" refer to signaling peptides. Cytokines are inhibitory or stimulatory. Chemokines are cytokines that induce chemotaxis of cells. Immune cells respond to chemokine gradients by directed movement to or away from the gradient. There are four classes of chemokines identified as CXC, CC, CX3C, and C. All four classes of chemokines interact with chemokine receptors expressed on the cell surface membranes of immune cells to guide the migration of the cells to the intended target. Typical chemokines are listed in Tables 1, 2, and 3.

A "cell surface receptor," as used herein, is a transmembrane protein that is found on the cell surface membrane of a cell. The cell surface receptor typically has an intracellular signaling domain, a transmembrane domain and an extracellular ligand binding domain and is capable of binding to another molecule, typically a signaling molecule such as a cytokine or a chemokine. A "chemokine cell surface receptor" is a cell surface receptor that belongs to the family of G protein-coupled receptors (GPCRs). The cognate ligands for chemokine receptors are generally chemokines. Chemokine receptors are typically found on the cell surface membrane of lymphocytes. GPCRs contain an N terminal extracellular ligand binding domain, seven transmembrane helices and a C terminal intracellular signaling domain. GPCRs typically bind one or more cognate ligands. After ligand binding, GPCRs typically recruit trimeric G proteins to the intracellular part. G protein recruitment typically induces a downstream signaling cascade. In lymphocytes, signaling cascades induced by chemokine receptor binding to cognate chemokines typically result in chemotaxis of the lymphocyte towards or away from the chemokine gradient. Exemplary chemokine receptors are listed in Tables 1, 2, and 3 below.

As used herein, "stem cell" refers to a cell that has the capacity for self-renewal, *i.e.,* the ability to go through numerous cycles of cell division while maintaining the undifferentiated state. Stem cells can be totipotent, pluripotent, multipotent, oligopotent, or unipotent. Stem cells are embryonic, fetal, amniotic, adult, or induced pluripotent stem cells.

As used herein, "induced pluripotent stem cells" refers to a type of pluripotent stem cell that is artificially derived from a non-pluripotent cell, typically an adult somatic cell, by inducing expression of specific genes.

As used herein, "hematopoietic stem cell" refers to an undifferentiated cell that has the ability to differentiate into a hematopoietic cell, such as a lymphocyte.

As used herein, the terms "epigenome," "epigenomic," and "epigenetic marker" include modifications to the genome of a cell, such as DNA methylation, histone modifications, DNA accessibility, and the like. The epigenome of a cell determines RNA and protein expression timing and levels. Although all T cells of one organism have the same genome (apart from the loci for the T cell receptors), each T cell subtype, subset, and differentiation state has a characteristic epigenome that results in a characteristic RNA and protein expression profile (*see, e.g.,* Durek et. al., Immunity (2016) 45:1148-1161). Typically, each T cell differentiation state has a characteristic set of cell surface receptors that are controlled by the epigenome and that can be used as phenotypic markers.

As used herein, "naive T cells" (Tn) refers to a subtype of T cells that have differentiated in bone marrow *in vivo,* and successfully undergone the central selection in the thymus. A single naive T cell is able to generate multiple subsets of memory T cells with different phenotypic and functional properties and different gene expression profiles. For example, these cells can further differentiate into stem cell memory T cells, central memory T cells, or effector memory T cells (all described further herein). Included within this subtype are the naive forms of helper T cells (CD4+) and cytotoxic T cells (CD8+). Naive T cells are commonly characterized by the surface expression of CD62L (L-selectin) and CCR7 (C-C chemokine receptor type 7); the absence of the activation markers CD25, CD44, or CD69; and the absence of the memory CD45RO isoform *(see, e.g.,* De Rosa et al., Nat. Med. (2001) 7:245-248; van den Broek et al., Nat. Rev. Immunol. (2018) 18:363-373). They also express functional IL-7 receptors, consisting of subunits IL-7 receptor-α, CD127, and common-γ chain CD132.

"Stem cell memory T cells" (Tscm) refers to a subtype of T cells that typically comprises 2%-3% of the circulating T cell pool *in vivo* and can be identified within a naive-like phenotype (CD45RA⁺CD45RO⁻CCR7⁺CD62L⁺CD27⁺CD28⁺) by expression of the memory marker CD95 *(see, e.g.,* Gattinoni et al., Nat. Med. (2011) 17: 1290-1297). Tscm cells can mount anamnestic responses and display gene transcript profiles encompassing features of both naive T cells and central memory T cells. Moreover, Tscm cells are multipotent progenitors that can both self-renew and differentiate *in vitro* and *in vivo* into the entire spectrum of memory T cells, including central memory T cells and effector memory T cells. *See, e.g.,* Ahmed et al., Cell Reports (2016) 17:2811-2812.

"Central memory T cells" (Tcm) refers to a subtype of T cells that express CD45RO, CCR7, and CD62L. Tcm cells also have intermediate to high expression of CD44, which can be used to distinguish Tn cells from Tcm cells. Included within this subtype are Tcm forms of helper T cells (CD4+) and cytotoxic T cells (CD8+). Tcm cells are commonly found in the lymph nodes and in the peripheral circulation. Tcm cells have the ability to self-renew due to high levels of phosphorylation of the transcription factor STATS. Tscm and Tcm cells are longer-lived and more proliferative than effector memory T cells or effector T cells.

"Effector memory T cells" (Tem) express CD45RO but lack expression of CCR7 and CD62L. They also have intermediate to high expression of CD44. CD62L acts as a "homing receptor" for lymphocytes to enter secondary lymphoid tissues. Thus, Tern cells are typically found in the peripheral circulation and tissues, rather than in the lymph nodes, and exhibit immediate effector function. In response to antigen stimulation, Tern cells proliferate and differentiate into CD62L⁻ effector T cells.

"Effector T cells" (Teff) are fully differentiated T cells. Effector T cells are short-lived cells, as opposed to memory cells which have a potential of long-term survival but have strong cytotoxic activity.

The terms "subject," "individual" or "patient" are used interchangeably herein and refer to any member of the phylum Chordata, including, without limitation, humans and other primates, including non-human primates such as rhesus macaques, chimpanzees and other monkey and ape species; farm animals, such as cattle, sheep, pigs, goats and horses; domestic mammals, such as dogs and cats; laboratory animals, including rabbits, mice, rats and guinea pigs; birds, including domestic, wild, and game birds, such as chickens, turkeys and other gallinaceous birds, ducks, and geese; and the like. The term does not denote a particular age or gender. Thus, the term includes adult, young, and newborn individuals as well as males and females. In some embodiments, a host cell is derived from a subject (e.g., lymphocytes, stem cells, progenitor cells, or tissue-specific cells). In some embodiments, the subject is a non-human subject.

The terms "effective amount" or "therapeutically effective amount" of a composition or agent, as provided herein, refer to a sufficient amount of the composition or agent to provide the desired response. Such responses will depend on the particular disease in question. For example, in cancers, a desired response includes, but is not limited to, reducing tumor size; reducing the amount or frequency of symptoms associated with the cancer in question; inhibiting progression of the cancerous state, such as by reducing or eliminating metastasis; slowing the typical rate of progression of the particular cancer; and the like. In the case of immune diseases, such as autoimmune diseases, the desired response is, for example, reduction or elimination of an inflammatory response, reduction of the amount or frequency of symptoms associated with the particular disorder, and the like. The exact amount required will vary from subject to subject, depending on the species, age, and general condition of the subject, the severity of the condition being treated, and the particular modified lymphocyte used, mode of administration, and the like. An appropriate "effective" amount in any individual case may be determined by one of ordinary skill in the art using routine experimentation.

"Treatment" or "treating" a particular disease includes: (1) preventing the disease, *i.e.* preventing the development of the disease or causing the disease to occur with less intensity in a subject that may be predisposed to the disease but does not yet experience or display symptoms of the disease, (2) inhibiting the disease, *i.e.,* reducing the rate of development, arresting the development or reversing the disease state, and/or (3) relieving symptoms of the disease *i.e.,* decreasing the number of symptoms experienced by the subject.

Clustered Regularly Interspaced Short Palindromic Repeats (CRISPR) and CRISPR-associated (Cas) proteins are programmable adaptive immune systems of bacterial and archaeal origin. After infection with a pathogenic virus, the bacterial or archaeal cell produces CRISPR-Cas effectors that destroy the foreign viral DNA or RNA by specifically recognizing target sequences and cleaving them.

CRISPR-Cas systems are classified into two distinct classes, Class 1 and Class 2 and are described in detail in Koonin et al., Curr Opin Microbiol. (2017) 37:67-78. Class 1 CRISPR-Cas systems comprise the multiprotein effector complexes (Type I (Cascade effector complex), III (Cmr/Csm effector complex), and IV), and Class 2 CRISPR-Cas systems comprise single effector proteins (Type II (Cas9), V (Cas12a, previously referred to as Cpf1), and VI (Cas13a, previously referred to as C2c2)). Additionally, CRISPR-Cas systems comprise one or more RNAs termed crRNAs or guide RNA that are derived from the CRISPRs. Some CRISPR-Cas systems comprise one or more RNAs termed tracrRNA derived from another locus. During an infection, CRISPR-Cas systems specifically incorporate foreign viral DNA or RNA sequences (spacers) into CRISPRs.

CRISPRs include repetitive elements that contain repeat-spacer-repeat subunits that are processed into crRNAs. A typical CRISPR can contain dozens to hundreds of repeat-spacer-repeats that were acquired in earlier infection rounds. Typically, for nucleic acid target recognition and cleavage, Class 1 and Class 2 effector proteins form an effector complex with one or more crRNAs comprising at least one spacer. Spacers that target nucleic acid sequences are typically complementary to the target region.

To distinguish between foreign and innate nucleic acids, some CRISPR-Cas systems recognize a protospacer adjacent motif (PAM) on the target nucleic acid. PAMs are variable in length, typically 3-6 bases, and are located 5' or 3' relative to the sequence complementary to the spacer. Many CRISPR-Cas systems are known in the art. By a "CRISPR-Cas system," as used herein, is meant any of the various CRISPR-Cas classes, types and subtypes.

As used herein, "a Cas protein" refers to a Cas protein derived from any species, subspecies, or strain of bacteria that encodes the Cas protein of interest, as well as variants and orthologs of the particular Cas protein in question. The Cas proteins and their cognate crRNAs or tracrRNAs can either be directly isolated and purified from bacteria, or synthetically or recombinantly produced, or can be delivered using a construct encoding the protein and a cognate NATNA, as defined herein, including without limitation, naked DNA, plasmid DNA, a viral vector, and mRNA for Cas expression. In the context of the present invention, Cas proteins are typically delivered to a host cell using a reconstituted Cas/NATNA complex or a plasmid that includes the desired *cas*/*NATNA* coding sequence.

The term "Cas9 protein," as used herein refers to wild-type proteins derived from Type II CRISPR-Cas9 systems, modifications of the Cas9 proteins, variants of Cas9 proteins, Cas9 orthologs, and combinations thereof. Cas9 proteins can be derived from any of various bacterial species having genomes that encode such proteins. Variants and modifications of Cas9 proteins are known in the art. U.S. Published Patent Application No. 2014/0273226, published 18 September 2014, discusses the *cas9* gene coding for the *Streptococcus pyogenes* Cas9 protein, termed "SpyCas9," the Cas9 protein, and variants of the Cas9 protein including host-specific codon-optimized Cas9 coding sequences and Cas9 fusion proteins. U.S. Published Patent Application No. 2014/0315985, published 23 October 2014, teaches a large number of exemplary wild-type Cas9 polypeptides including the sequence of SpyCas9. Modifications and variants of Cas9 proteins are also discussed. Non-limiting examples of Cas9 proteins include Cas9 proteins from *S. pyogenes* (GI:15675041); *Listeria innocua* Clip 11262 (GI:16801805); *Streptococcus mutans* UA159 (GI:24379809); *Streptococcus thermophilus* LMD-9 (S. *thermophilus* A, GI: 11662823; *S. thermophilus* B, GI: 116627542); *Lactobacillus buchneri* NRRL B-30929 (GI:331702228); *Treponema denticola* ATCC 35405 (GI:42525843); *Francisella novicida* U112 (GI:118497352); *Campylobacter jejuni* subsp. *Jejuni* NCTC 11168 (GI:218563121); *Pasteurella multocida* subsp. *multocida* str. Pm70 (GI:218767588); *Neisseria meningitidis* Zs491 (GI:15602992); *and Actinomyces naeslundii* (GI:489880078).

By "dCas protein" is meant a nuclease-deactivated Cas protein, also termed "catalytically inactive," "catalytically dead" or "dead Cas protein." Such molecules lack all or a portion of endonuclease activity and can therefore be used to regulate genes in an RNA-guided manner (Jinek et al., Science (2012) 337:816-821). This is accomplished by introducing mutations that inactivate Cas nuclease function. For Cas9, this is typically accomplished by mutating both of the two catalytic residues (D10A in the RuvC-1 domain, and H840A in the HNH domain, numbered relative to SpyCas9) of the gene encoding Cas9. It is understood that mutation of other catalytic residues to reduce activity of either or both of the nuclease domains can also be carried out by one skilled in the art. In doing so, dCas9 is unable to cleave dsDNA but retains the ability to sequence-specifically bind DNA. The Cas9 double mutant with changes at amino acid positions D10A and H840A completely inactivates both the nuclease and nickase activities. Targeting specificity is determined by complementary base-pairing of a single guide RNA to the genomic locus and the protospacer adjacent motif (PAM).

A Cas9 dual-guide RNA (Cas9-dgRNA) is a guide RNA comprising a Cas9-crRNA and a Cas9-tracrRNA that form a duplex RNA structure described in Jinek et al., Science (2012) 337:816-821. Cas9 complexed with dgRNA can bind and cleave DNA complementary to the crRNA sequence.

A Cas9 single-guide RNA (Cas9-sgRNA) is a guide RNA wherein the Cas9-crRNA is covalently joined to the Cas9-tracrRNA, often through a tetraloop, and forms a RNA polynucleotide secondary structure through base-pair hydrogen bonding as described in Jinek et al., Science (2012) 337:816-821 and U.S. Published Patent Application No. 2014/0068797, published 6 March 2014.

A "nucleic acid-targeting nucleic acid" (NATNA), also known as a "guide polynucleotide," refers to one or more polynucleotides that guide a protein, such as a Cas9, or a deactivated Cas endonuclease, or a polynucleotide binding domain that is part of an artificial transcriptional activator complex as described herein, to preferentially target a nucleic acid target sequence present in a polynucleotide (relative to a polynucleotide that does not comprise the nucleic acid target sequence). NATNAs can comprise ribonucleotide bases (*e*.*g*., RNA), deoxyribonucleotide bases (*e*.*g*., DNA), combinations of ribonucleotide bases and deoxyribonucleotide bases (e.g., RNA/DNA), nucleotides, nucleotide analogs, modified nucleotides, and the like, as well as synthetic, naturally occurring, and non-naturally occurring modified backbone residues or linkages. Thus, a NATNA, as used herein site-specifically guides a protein, such as Cas9, or an activator complex that includes an activation domain as defined herein, to a target nucleic acid. Many such NATNAs are known, such as but not limited to sgNATNAs (including miniature and truncated single-guide NATNAs as well as crRNA molecules); dual-guide NATNAs, including but not limited to, crRNA/tracrRNA molecules; and the like. For a non-limiting description of exemplary NATNAs, *see, e.g.,* PCT Publication No. WO 2014/150624, published 29 September 2014; PCT Publication No. WO 2015/200555, published 10 March 2016; PCT Publication No. WO 2016/201155, published 15 December 2016; PCT Publication No. WO 2017/027423, published 16 February 2017; PCT Publication No. WO 2017/070598, published 27 April 2017; and PCT Publication No. WO 2016/123230, published 4 August 2016.

The terms as used herein also intend a guide domain present within a zinc finger DNA-binding domain, a Transcription activator-like (TAL) effector DNA binding domain, and the like, that guides a non-CRISPR nucleic acid binding domain to a selected site to bind the site.

As used herein, a molecule is said to "target" a polynucleotide if the polynucleotide binding domain in an artificial transcriptional activator complex associates with and binds to a polynucleotide at the nucleic acid target sequence within the polynucleotide.

As used herein, a "site-directed polypeptide or protein" refers to a polypeptide that recognizes and/or binds to a nucleic acid target sequence or the complement of the nucleic acid target sequence. The site directed polypeptide, alone or in combination with polynucleotides such as NATNAs, will bind to a nucleic acid target sequence or to the complement of the nucleic acid target sequence. Optionally, the site-directed polypeptide provides amino acid sequences that recruit endogenous transcription factors to the nucleic acid target sequence. Optionally, the NATNA provides nucleic acid sequences that recruit endogenous transcription factors to the nucleic acid target sequence.

As used herein, the term "cognate" refers to biomolecules that interact, such as a cell surface receptor (*e.g.,* a chemokine receptor), and its ligand (*e.g.,* a chemokine expressed on a tumor cell or in a tumor microenvironment); a site-directed polypeptide and its NATNA; a site-directed polypeptide/NATNA complex (*i.e*., a nucleoprotein complex) capable of site-directed binding to a nucleic acid target sequence complementary to the NATNA binding sequence; and the like.

As used herein, the terms "effector complex," "complex," "nucleoprotein complex," "NATNA/Cas9 complex," "NATNA/dCas9 complex," "Cas protein/NATNA nucleoprotein complex," and "crRNA-effector complex," refer to complexes comprising a NATNA and a protein that binds to a nucleic acid target sequence. The Cas protein of the complex can effect a blunt-ended double-strand break, a double-strand break with sticky ends, nick one strand, or perform other functions on the nucleic acid target sequence. In the context of the present invention, the complex typically includes a catalytically inactive protein that binds but does not cleave the targeted nucleic acid.

"CRISPRa" (CRISPR activation) is a CRISPR method or system wherein the method or system activates the expression of a gene within the locus of the target nucleic acid sequence. In the context of the present invention, CRISPRa typically utilizes a catalytically inactive Cas protein, such as a dCas9, complexed with a NATNA, such as a sgRNA or dgRNA. For the recruitment of endogenous transcription factors, the dCas9 protein or the NATNA in the complex is fused to an effector domain such as, but not limited to, VP16 or VP64 (Eguchi et al., Proc. Natl. Acad. Sci. USA (2016) 113:E8257-E8266; Perez-Pinera et al., Nature Methods (2013) 10:973-976; Gilbert et al. Cell (2014) 159:647-661), or an NFkappaB p65 domain or an Epstein Barr Virus R protein (BRLF1). A linker sequence can be present between the Cas protein or the NATNA, and the effector domain. Alternatively, the NATNA can be fused to a nucleotide effector domain such as an MS2 binding RNA that recruits transcription factors (described, *e*.*g*., in Konermann et al., Nature (2015) 517:583-588). Effector domain fusion to dCas9 and the NATNA can also be combined (Mali et al. Nature Biotechnology (2013) 31:833-838) to activate expression of the endogenous gene. The target locus contains one or more transcriptional start sites (TSSs) that typically harbors a binding site for the transcriptional activation machinery (factors) of a cell.

"CRISPRi" (CRISPR inhibition) is a CRISPR method or system wherein the CRISPR method or system downregulates the expression of a gene within the locus of the target nucleic acid sequence. In the context of the present invention, CRISPRi typically utilizes a catalytically inactive Cas protein, such as a dCas9, complexed with a NATNA, such as a sgRNA, to downregulate the expression of the gene. Alternatively, the dCas9 protein can be fused to a transcriptional repressor such as KRAB that will recruit additional endogenous transcriptional repression effector proteins that downregulate the expression of the gene.

"CASCADEa" (CASCADE activation) is a CRISPR method or system wherein the method or system activates the expression of a gene within the locus of the target nucleic acid sequence. For the recruitment of endogenous transcription factors, one or more proteins in the CASCADE complex or the NATNA, is typically fused to an effector domain such as VP16 or VP64. For a description of the CASCADE complex *see, e.g.,* Jore et al., Nature Structural and Molecular Biology (2011) 18:529-536. Alternatively, the NATNA can be fused 5' or 3' to a nucleotide effector domain such as an MS2 binding RNA that also recruits transcription factors. Effector domain fusion to a CASCADE protein and the NATNA can also be combined. The target locus contains one or more transcriptional start sites (TSSs) that typically harbors a binding site for the transcriptional activation machinery (factors) of a cell.

"CASCADEi" (CASCADE inhibition) is a CRISPR method or system wherein the CRISPR method or system downregulates the expression of a gene within the locus of the target nucleic acid sequence. CASCADEi typically utilizes the CASCADE complex with a NATNA to downregulate the expression of the gene. Alternatively, the proteins in the CASCADE protein can be fused to a transcriptional repressor such as KRAB that will recruit additional endogenous transcriptional repression effector proteins that downregulate the expression of the gene.

"Transcription activation-like effectors" (TALEs) are DNA binding proteins of bacterial origin. The TAL effector DNA-binding domain recognizes specific individual base pairs in a target DNA sequence by using a known cipher involving two key amino acid residues, also referred to as the repeat variable di-residues (RVDs). *See, e.g.,* Mussolino et al., Nucleic Acids Res. (2011) 39:9283-9293. Depending on the TALE protein sequence, TALEs can bind any DNA base (G, T, A, C). A large number of TALEs are known in the art. Several TALE DNA binding domains can be fused together and engineered to bind any contiguous DNA sequence. Typically, about 15 TALE DNA binding domains are fused together to recognize a 15 nucleotide long DNA sequence. TALEs can be fused to transcriptional activators and repressors. Engineered TALEs can be used for transcriptional activation or repression in a cell, such as a lymphocyte.

"TALEa" (TALE activation) is a method or system wherein the method or system activates the expression of a gene by using one or more TALEs. For the recruitment of endogenous transcription factors, the TALE is typically fused to an effector domain such as VP16 or VP64. This complex targets and binds to a nucleic acid sequence within the locus of the genome and activates expression of the gene. The target locus contains one or more transcriptional start sites (TSSs) that typically harbors a binding site for the transcriptional activation machinery (factors) of a cell, such as a lymphocyte.

"TALEi" (TALE inhibition) typically utilizes the TALE to downregulate the expression of the gene. Alternatively, the TALE can be fused to a transcriptional repressor such as KRAB that will recruit additional endogenous transcriptional repression effector proteins that downregulate the expression of the gene.

Transcription activation-like effector nucleases (TALENs) are TALEs that are fused to the DNA-cleaving domain of a restriction enzyme such as FokI. TALENs are engineered to bind and cleave any desired DNA sequence. TALENs are typically used for genome engineering of an organism.

"Meganucleases" or "homing endonucleases" refer to a family of enzymes that recognize, bind, and cleave specific DNA sequences (reviewed in Stoddard, B, Mobile DNA (2014) 5:7. The DNA recognition site of meganucleases are typically 12 to 40 base pairs. A large number of meganucleases are known in the art. Meganucleases can be engineered to bind and cleave any DNA sequence. In the context of the present invention, meganucleases are engineered such that they are catalytically inactive and can bind but not cleave DNA. Meganucleases can be fused to other proteins such as transcriptional activators and repressors or other nucleases. Engineered meganucleases can be used for transcriptional activation or repression or genome engineering of a cell, such as a lymphocyte.

"Meganuclease a" (meganuclease activation) is a method or system wherein expression of a gene is activated by using one or more meganucleases that bind to a genomic sequence. In the context of the present invention, the meganuclease is typically catalytically inactive. For the recruitment of endogenous transcription factors, the meganuclease is fused to an effector domain such as VP16 or VP64. The meganuclease a system targets and binds to a nucleic acid sequence within the locus of the genome and activates expression of the gene. The target locus contains one or more transcriptional start sites (TSSs) that harbors a binding site for the transcriptional activation machinery (factors) of a cell, such as a lymphocyte.

"Meganuclease i" (meganuclease inhibition) utilizes the meganuclease to downregulate expression of the gene. Alternatively, the meganuclease can be fused to a transcriptional repressor such as KRAB that will recruit additional endogenous transcriptional repression effector proteins that downregulate the expression of the gene.

"Zinc fingers" (ZnFs) are DNA binding proteins or DNA binding protein domains. The proteins or protein domains are often but not always coordinated with one or more zinc ions that recognize particular DNA sequences. A large number of ZnF domains and proteins are known in the art. Depending on the ZnF sequence, one ZnF domain typically binds a triplet of DNA bases. Several ZnFs can be fused together and engineered to bind any target DNA sequence. Generally, about 5 ZnF DNA binding domains are fused together to recognize a 15 nucleotide long DNA sequence. ZnFs can be fused to transcriptional activators and repressors. Engineered ZnF can be used for transcriptional activation or repression in a cell, such as a lymphocyte.

ZnF nucleases are engineered ZnFs that are fused with the DNA-cleaving domain of a restriction enzyme such as FokI. ZnF nucleases can be engineered to bind and cleave any target DNA sequence. Engineered ZnF nucleases are typically used for genome engineering of an organism.

"ZnFa" (zinc finger activation) is a method or system wherein expression of a gene is activated by using one or more zinc finger proteins that bind to a genomic sequence. *See, e.g.,* Ji et al., Nucleic Acids Res. (2014) 42:6158-6167. For the recruitment of endogenous transcription factors, the ZnF is typically fused to an effector domain such as VP16 or VP64. The complex targets and binds to a nucleic acid sequence within the locus of the genome and activates expression of the gene. The target locus contains one or more transcriptional start sites (TSSs) that harbors a binding site for the transcriptional activation machinery (factors) of a cell.

"ZnFi" (zinc finger inhibition) typically utilizes the ZnF to downregulate the expression of the gene. Alternatively, the ZnF can be fused to a transcriptional repressor such as KRAB that will recruit additional endogenous transcriptional repression effector proteins that downregulate the expression of the gene.

The terms "wild-type," "naturally occurring" and "unmodified" are used herein to mean the typical (or most common) form, appearance, phenotype, or strain existing in nature; for example, the typical form of cells, organisms, characteristics, polynucleotides, proteins, macromolecular complexes, genes, RNAs, DNAs, or genomes as they occur in and can be isolated from a source in nature. The wild-type form, appearance, phenotype, or strain serve as the original parent before an intentional modification. Thus, mutant, variant, chimeric, engineered, recombinant, and modified forms are not wild-type forms.

As used herein, the terms "engineered," "genetically engineered," "recombinant," "modified," and "non-naturally occurring" are interchangeable and indicate intentional human manipulation.

As used herein, the terms "nucleic acid," "nucleotide sequence," "oligonucleotide," and "polynucleotide" are interchangeable. All refer to a polymeric form of nucleotides. The nucleotides may be deoxyribonucleotides (DNA) or ribonucleotides (RNA), combinations thereof or analogs thereof, and they may be of any length. Polynucleotides may perform any function and may have any secondary structure and three-dimensional structure. The terms encompass known analogs of natural nucleotides and nucleotides that are modified in the base, sugar and/or phosphate moieties. Analogs of a particular nucleotide have the same base-pairing specificity (e.g., an analog of A base pairs with T). A polynucleotide may comprise one modified nucleotide or multiple modified nucleotides. Examples of modified nucleotides include methylated nucleotides and nucleotide analogs. Nucleotide structure may be modified before or after a polymer is assembled. Following polymerization, polynucleotides may be additionally modified via, for example, conjugation with a labeling component or target-binding component. A nucleotide sequence may incorporate non-nucleotide components. The terms also encompass nucleic acids comprising modified backbone residues or linkages that (i) are synthetic, naturally occurring, and non-naturally occurring, and (ii) have similar binding properties as a reference polynucleotide (e.g., DNA or RNA). Examples of such analogs include, but are not limited to, phosphorothioates, phosphoramidates, methyl phosphonates, chiral-methyl phosphonates, 2-O-methyl ribonucleotides, peptide-nucleic acids (PNAs), and morpholino structures.

Polynucleotide sequences are displayed herein in the conventional 5' to 3' orientation.

As used herein, the term "complementarity" refers to the ability of a nucleic acid sequence to form hydrogen bond(s) with another nucleic acid sequence (*e*.*g*., through traditional Watson-Crick base pairing, Hoogsteen hydrogen bonding or wobble hydrogen bonding). A percent complementarity indicates the percentage of residues in a nucleic acid molecule that can form hydrogen bonds with a second nucleic acid sequence. When two polynucleotide sequences have 100% complementarity, the two sequences are perfectly complementary, *i.e.,* all of a first polynucleotide's contiguous residues hydrogen bond with the same number of contiguous residues in a second polynucleotide.

As used herein, the term "sequence identity" generally refers to the percent identity of bases or amino acids determined by comparing a first polynucleotide or polypeptide to a second polynucleotide or polypeptide using algorithms having various weighting parameters. Sequence identity between two polypeptides or two polynucleotides can be determined using sequence alignment by various methods and computer programs (*e*.*g*., CLUSTAL Omega, Jalview, BLAST, CS-BLAST, FASTA, HMMER, L-ALIGN, etc.), available through the worldwide web at sites including GENBANK (ncbi.nlm.nih.gov/genbank/) and EMBL-EBI (ebi.ac.uk.). Sequence identity between two polynucleotides or two polypeptide sequences is generally calculated using the standard default parameters of the various methods or computer programs. Generally, the various proteins for use herein will have at least about 75% or more sequence identity to the wild-type or naturally occurring sequence of the protein of interest, such as about 80%, such as about 85%, 90%, 91%, 92%, 93%, 94%, 95%, 96%, 97%, 98%, 99%, or complete identity.

As used herein, "double-strand break" (DSB) refers to both strands of a double-stranded segment of nucleic acid being severed. In some instances, if such a break occurs, one strand can be said to have a "sticky end" wherein nucleotides are exposed and not hydrogen bonded to nucleotides on the other strand. In other instances, a "blunt end" can occur wherein both strands remain fully base paired with each other.

The terms "vector" and "plasmid" are used interchangeably and refer to a polynucleotide vehicle to introduce genetic material into a cell. Vectors can be linear or circular. Vectors can integrate into a target genome of a host cell or replicate independently in a host cell. Vectors can comprise, for example, an origin of replication, a multicloning site, and/or a selectable marker. An expression vector typically comprises an expression cassette. Vectors and plasmids include, but are not limited to, integrating vectors, prokaryotic plasmids, eukaryotic plasmids, plant synthetic chromosomes, episomes, viral vectors, cosmids, and artificial chromosomes. In the context of the present invention, a vector can comprise a sequence for a NATNA, a sequence for a protein capable of binding the genomic region recognized by the NATNA when complexed with the NATNA, and/or a sequence for an activation domain that upregulates or downregulates transcription, for example, of an endogenous cell surface receptor gene in a lymphocyte. Vectors can also include sequences encoding selectable or screenable markers, as well as polynucleotides encoding protein tags (*e*.*g*., poly-His tags, hemagglutinin tags, fluorescent protein tags, and bioluminescent tags). The coding sequences for such protein tags are fused to, for example, one or more nucleic acid sequences encoding a Cas protein.

"Gene," as used herein, refers to a polynucleotide sequence comprising exon(s) and related regulatory sequences. A gene may further comprise intron(s) and/or untranslated region(s) (UTR(s)).

As used herein, the term "expression" refers to transcription of a polynucleotide from a DNA template, resulting in, for example, an mRNA or other RNA transcript (*e*.*g*., non-coding, such as structural or scaffolding RNAs). The term further refers to the process through which transcribed mRNA is translated into peptides, polypeptides, or proteins. Transcripts and encoded polypeptides may be referred to collectively as "gene product." Expression may include splicing the mRNA in a eukaryotic cell, if the polynucleotide is derived from genomic DNA.

As used herein, the term "expression cassette" is a polynucleotide construct, generated recombinantly or synthetically, comprising regulatory sequences operably linked to a selected polynucleotide to facilitate expression of the selected polynucleotide in a host cell. For example, the regulatory sequences can facilitate transcription of the selected polynucleotide in a host cell, or transcription and translation of the selected polynucleotide in a host cell. An expression cassette can, for example, be integrated in the genome of a host cell or be present in an expression vector.

As used herein, the terms "regulatory sequences," "regulatory elements," and "control elements" are interchangeable and refer to polynucleotide sequences that are upstream (5' non-coding sequences), within, or downstream (3' non-translated sequences) of a polynucleotide sequence to be expressed. Regulatory sequences influence, for example, the timing of transcription, amount or level of transcription, RNA processing or stability, and/or translation of the related structural nucleotide sequence. Regulatory sequences may include activator binding sequences, enhancers, introns, polyadenylation recognition sequences such as from bovine growth hormone (BGH) or Simian Virus 40 (SV40), promoters, repressor binding sequences, stem-loop structures, translational initiation sequences, translation leader sequences, transcription termination sequences, translation termination sequences, primer binding sites, and the like.

As used herein, the term "operably linked" refers to polynucleotide sequences or amino acid sequences placed into a functional relationship with one another. For instance, a promoter or enhancer is operably linked to a coding sequence if it regulates, or contributes to the modulation of, the transcription of the coding sequence. Operably linked DNA sequences encoding regulatory sequences are typically contiguous to the coding sequence. However, enhancers can function when separated from a promoter by up to several kilobases or more. Additionally, multicistronic constructs can include multiple coding sequences that use only one promoter by including a 2A self-cleaving peptide, an IRES element, etc. Accordingly, some polynucleotide elements may be operably linked but not contiguous.

As used herein, the term "amino acid" refers to natural and synthetic (unnatural) amino acids, including amino acid analogs, modified amino acids, peptidomimetics, glycine, and D or L optical isomers.

As used herein, the terms "peptide," "polypeptide," and "protein" are interchangeable and refer to polymers of amino acids. A polypeptide may be of any length. It may be branched or linear, it may be interrupted by non-amino acids, and it may comprise modified amino acids. The terms may be used to refer to an amino acid polymer that has been modified through, for example, acetylation, disulfide bond formation, glycosylation, lipidation, phosphorylation, crosslinking, and/or conjugation (*e*.*g*., with a labeling component or ligand). Polypeptide sequences are displayed herein in the conventional N-terminal to C-terminal orientation.

Polypeptides and polynucleotides can be made using routine techniques in the field of molecular biology (*see, e.g.,* standard texts discussed above). Furthermore, essentially any polypeptide or polynucleotide can be custom ordered from commercial sources.

The term "binding," as used herein includes a non-covalent interaction between macromolecules (*e*.*g*., between a protein and a polynucleotide, between a polynucleotide and a polynucleotide, or between a protein and a protein, and the like). Such non-covalent interactions are also referred to as "associating" or "interacting" (*e*.*g*., if a first macromolecule interacts with a second macromolecule, the first macromolecule binds to second macromolecule in a non-covalent manner). Some portions of a binding interaction may be sequence-specific. The terms "sequence-specific binding," "sequence-specifically bind," "site-specific binding," and "site specifically binds" are used interchangeably herein. Sequence-specific binding, as used herein, typically refers to a nucleic acid binding protein that binds a nucleic acid sequence (*e*.*g*., a DNA sequence) comprising a nucleic acid target sequence (*e*.*g*., a DNA target sequence) preferentially relative to a second nucleic acid sequence (*e.g.,* a second DNA sequence) without the nucleic acid target binding sequence (*e.g.,* the DNA target binding sequence), or refers to one or more NATNAs capable of forming a complex with a protein to form a nucleoprotein complex, to cause the complex to bind a nucleic acid sequence (*e.g.,* a DNA sequence) comprising a nucleic acid target sequence (*e*.*g****.*,** a DNA target sequence) preferentially relative to a second nucleic acid sequence *(e.g.,* a second DNA sequence) without the nucleic acid target binding sequence (*e.g.,* the DNA target binding sequence). All components of a binding interaction do not need to be sequence-specific, such as contacts of a protein with phosphate residues in a DNA backbone. Binding interactions can be characterized by a dissociation constant (Kd). "Binding affinity" refers to the strength of the binding interaction. An increased binding affinity is correlated with a lower Kd.

As used herein, the term "isolated" can refer to a nucleic acid or polypeptide that, by the hand of a human, exists apart from its native environment and is therefore not a product of nature. Isolated means substantially pure. An isolated nucleic acid or polypeptide can exist in a purified form and/or can exist in a non-native environment such as, for example, in a recombinant cell.

As used herein, a "host cell" generally refers to a biological cell. A cell can be the basic structural, functional and/or biological unit of a living organism. A cell can originate from any organism having one or more cells. Examples of host cells include, but are not limited to: a prokaryotic cell, a eukaryotic cell, a bacterial cell, an archaeal cell, a cell of a single-cell eukaryotic organism, a protozoa cell, a cell from a plant, a fungal cell (*e.g.,* a yeast cell, a cell from a mushroom), an insect cell, an animal cell, a cell from an invertebrate animal (*e.g.* fruit fly, cnidarian, echinoderm, nematode, etc.), a cell from a vertebrate animal (*e.g*., fish, amphibian, reptile, bird, mammal), and a cell from a mammal (*e*.*g*., a pig, a cow, a goat, a sheep, a rodent, a rat, a mouse, a non-human primate, a human, etc.). Furthermore, a cell can be a stem cell or progenitor cell.

The present invention is directed to lymphocytes that have been modified to ectopically express endogenous chemokine receptor genes that are normally silent. The lymphocytes are modified such that chemokine receptor genes are expressed on the cell surface and can therefore target their cognate chemokines present on, or secreted by, cancerous cells, such as tumor cells or by the tumor microenvironment. This allows the modified lymphocytes to home-in, attack, and kill these cells and provides an effective treatment for various cancers. Current methods for modifying lymphocytes to express cell surface chemokine receptors include editing the lymphocyte genome to insert an expression cassette that expresses the chemokine receptor using recombinant DNA technology or CRISPR gene editing techniques. In contrast, the present invention activates endogenous genes that are not normally expressed in the lymphocyte in question due to endogenous repressors. Thus, the modification of the present invention does not comprise an engineered insertion of the chemokine receptor-encoding gene into the lymphocyte cell genome, such as by the use of gene editing techniques including recombinant DNA technology and CRISPR-Cas methods.

Lymphocytes for use in the present invention are human T cells including T cells for cell-mediated, cytotoxic adaptive immunity, such as CD4+ and/or CD8+ cytotoxic T cells; Additionally, CAR-T cells, TCR cells, including TCR engineered CAR-T cells, TILs, CAR TILs, and the like, can be modified using the techniques herein.

Lymphocytes for modification can be isolated from a human subject, for example from blood or from solid tumors, such as in the case of TILs, or from lymphoid organs such as the thymus, bone marrow, lymph nodes, and mucosal-associated lymphoid tissues. Techniques for isolating lymphocytes are well known in the art. For example, lymphocytes can be isolated from peripheral blood mononuclear cells (PBMCs), which are separated from whole blood using, for example, ficoll, a hydrophilic polysaccharide that separates layers of blood, and density gradient centrifugation. Generally, anticoagulant or defibrinated blood specimens are layered on top of a ficoll solution, and centrifuged to form different layers of cells. The bottom layer includes red blood cells (erythrocytes), which are collected or aggregated by the ficoll medium and sink completely through to the bottom. The next layer contains primarily granulocytes, which also migrate down through the ficoll-paque solution. The next layer includes lymphocytes, which are typically at the interface between the plasma and the ficoll solution, along with monocytes and platelets. To isolate the lymphocytes, this layer is recovered, washed with a salt solution to remove platelets, ficoll and plasma, then centrifuged again.

Other techniques for isolating lymphocytes include biopanning, which isolates cell populations from solution by binding cells of interest to antibody-coated plastic surfaces. Unwanted cells are then removed by treatment with specific antibody and complement. Additionally, fluorescence activated cell sorter (FACS) analysis can be used to detect and count lymphocytes. FACS analysis uses a flow cytometer that separates labelled cells based on differences in light scattering and fluorescence.

For TILs, lymphocytes are isolated from a tumor and grown, for example, in high-dose IL-2 and selected using cytokine release coculture assays against either autologous tumor or HLA-matched tumor cell lines. Cultures with evidence of increased specific reactivity compared to allogeneic non-MHC matched controls are selected for rapid expansion and then introduced into a subject in order to treat cancer. *See, e.g.,* Rosenberg et al., Clin. Cancer Res. (2011) 17:4550-4557; Dudly et al., Science (2002) 298:850-854; Dudly et al., J. Clin. Oncol. (2008) 26:5233-5239; Dudley et al., J. Immnother. (2003) 26:332-342.

Upon isolation, lymphocytes can be characterized in terms of specificity, frequency and function. Frequently used assays include an ELISPOT assay, which measures the frequency of T cell response.

In some embodiments, lymphocytes for modification are isolated from a subject, modified *in vitro,* and then reintroduced into the same subject. This technique is known as autologous lymphocyte therapy. Alternatively, lymphocytes can be isolated, modified *in vitro,* and introduced into a different subject. This technique is known as allogenic lymphocyte therapy.

After isolation, lymphocytes can be activated using techniques well known in the art in order to promote proliferation and differentiation into specialized effector lymphocytes. Surface markers for activated T cells include, for example, CD3, CD4, CD8, PD1, IL2R, and others. Activated cytotoxic lymphocytes can kill target cells after binding cognate receptors on the surface of target cells. Surface markers for NK cells include, for example CD16, CD56, and others.

Following isolation and optionally activation, lymphocytes are modified in order to express one or more endogenous chemokine receptor genes present in the lymphocyte genome that are normally epigenetically silenced. When expressed, the chemokine receptor presents on the lymphocyte cell surface and is specific for, and targets, the lymphocyte to a cognate chemokine present on the tumor cell surface or secreted by the tumor microenvironment.

Lymphocytes according to the invention are modified by delivering an artificial transcription factor (ATF) complex in proximity to the transcriptional start site (TSS) of the desired endogenous chemokine receptor-encoding gene. The TSS includes a binding site for the transcriptional activation machinery of the lymphocyte in order to turn on expression of the desired gene. The ATF complex includes at least two components, a polynucleotide binding domain that recognizes cognate nucleotide sequences, and an activation domain (also known as an effector domain) that recruits the transcriptional activation machinery in order to ectopically express the endogenous gene. The polynucleotide binding domain portion of the ATF includes a nucleoprotein complex that comprises a NATNA, to guide a site-directed protein to the region of interest.

Non-limiting examples of effector domains include CRISPR-associated protein transcription factors such as found in CRISPRa and CASCADEa; zinc finger transcription factors, such as found in ZnFa; TALE transcription factors, such as found in TALEa; meganuclease transcription factors; and various small molecules, antibodies, polypeptides or oligonucleotides described herein. Such effector domains, can be from, without limitation, VP16, VP64, NFkappaB p65 domain, or Epstein Barr Virus R protein (BRLF1). Alternatively, the effector domain can be an MS2-binding RNA that recruits transcription factors.

In one embodiment, an ATF complex is introduced into a lymphocyte. The ATF complex includes a site-directed DNA binding protein, a NATNA, and a transcriptional activator. The DNA binding protein is directed to a site in proximity to the TSS by the NATNA, binds the site, and the transcriptional activator recruits transcription factors to the TSS so that the endogenous gene is expressed. The components of the ATF complex can be delivered together, in a single construct or as a ribonucleoprotein particle or, for example, one construct can be delivered that includes a nucleoprotein, *e*.*g*., a DNA site-directed binding protein fused to the transcriptional activator, and a second construct can include the NATNA. If the NATNA and the binding protein/transcriptional activator fusion are delivered separately, the nucleoprotein will form a complex with the NATNA in the lymphocyte and will therefore be delivered to a region in proximity of the TSS to turn on transcription of the endogenous chemokine receptor gene.

In some embodiments, the NATNA is associated either directly or indirectly, *e*.*g*., by a linker sequence 5' or 3' or internally, with the MS2 binding RNA sequence that recruits the transcriptional activator domain. In other embodiments, the site-directed binding domain protein is associated either directly or indirectly with the transcriptional activator domain. Thus, in the complexed ATF, the activator domain can be located either 5' or 3' or internally of the NATNA/site-directed protein complex.

The site-directed protein is a Cas protein, such as a Cas9 protein that has been engineered to be catalytically inactive (dCas) so that the protein binds to a nucleic acid target region in a lymphocyte genome when in association with a cognate NATNA, but does not cleave the genome. In such embodiments, the Cas protein, such as dCas9, is directed in proximity to the TSS using a guide polynucleotide, such as a sgRNA or a dgRNA. Methods of making ATF complexes using a dCas9 protein, sgRNA and, *e.g.,* VP64, are described herein and known in the art. See, *e.g.,* Mali et al., Nature Biotech. (2013) 31:230-232. In some embodiments, the VP64 transcriptional activation domain can be directly tethered to the C-terminus of dCas9. Alternatively, VP64 can be tethered to an aptamer-modified sgRNA, as described in Mali et al., Nature Biotech. (2013) 31:230-232. In this embodiment, sgRNA tethers capable of recruiting activation domains can be generated by appending one or more copies of the MS2 bacteriophage coat protein-binding RNA stem-loop to the 3'-end of the sgRNA and the chimeric sgRNAs can be expressed together with dCas9 and the MS2-VP64 fusion protein, or can be directly delivered as a complex to the cell.

After producing the modified lymphocytes, the lymphocytes are screened to select for cells expressing the desired cell surface receptor, using methods such as high-throughput screening techniques including, but not limited to, fluorescence-activated cell sorting (FACS)-based screening platforms, microfluidics-based screening platforms, and the like. These techniques are well known in the art and reviewed in, for example, Wojcik et al., Int. J. Molec. Sci. (2015) 16:24918-24945 and described in Example 4 herein.

Using the methods described herein, lymphocytes can be modified to express any number of endogenous chemokine receptor-encoding genes. According to the invention, the endogenous chemokine receptor expressed by the modified lymphocyte is selected from the group consisting of CCR1, CCR2, CCR3, CCR4, CCR5, CXCR4, or DARC. Tables 1 and 2 provide lists of exemplary chemokine receptors and their cognate chemokines. In Table 1, the SEQ ID NOS are the sequences of the chemokines. In Table 2, the SEQ ID NOS are the sequences of the chemokine receptors.

**Table 1: List of exemplary chemokines and their cognate chemokine receptors**

| **Chemokine** | **Cognate chemokine receptor(s)** | **SEQ ID NO:** |
|---|---|---|
| CCL2 | CCR2 | SEQ ID NO: 1 |
| CCL3 | CCR5 | SEQ ID NO: 2 |
| CCL4 | CCR5 | SEQ ID NO: 3 |
| CCL5 | CCR1, CCR3, CCR4, CCR5, DARC, CXCR3 | SEQ ID NO: 4 |
| CXCL8 | CXCR1 | SEQ ID NO: 5 |
| CXCL10 | CXCR3 | SEQ ID NO: 6 |
| CXCL12 | CXCR4 | SEQ ID NO: 7 |
| CCL17 (TARC) | CCR4, DARC | SEQ ID NO: 8 |

**Table 2: List of exemplary chemokine receptors and their cognate chemokines**

| **Chemokine receptor** | **Cognate chemokine(s)** | **SEQ ID NO:** |
|---|---|---|
| CCR1 | CCL5 | SEQ ID NO: 9 |
| CCR2 | CCL2 | SEQ ID NO: 10 |
| CCR3 | CCL5 | SEQ ID NO: 11 |
| CCR4 | CCL5, CCL17 | SEQ ID NO: 12 |
| CCR5 | CCL5, CCL3, CCL4 | SEQ ID NO: 13 |
| CXCR1 | CXCL8 | SEQ ID NO: 14 |
| CXCR3 | CXCL10, CCL5 | SEQ ID NO: 15 |
| CXCR4 | CXCL12 | SEQ ID NO: 16 |
| DARC | CCL17, CCL5 | SEQ ID NO: 17 |

One embodiment of the invention provides modified lymphocytes that are targeted to cancerous cells from various types of cancers. Such cancers include, without limitation, prostate cancers; ovarian cancers; cervical cancers; colorectal cancers; intestinal cancers; testicular cancers; skin cancers; lung cancers; thyroid cancers; bone cancers; breast cancers; bladder cancers; uterine cancers; vaginal cancers; pancreatic cancers; liver cancers; kidney cancers; brain cancers; spinal cord cancers; oral cancers; parotid tumors; blood cancers; lymphomas, solid tumors, liquid tumors, etc. Table 3 describes exemplary tumor types and the chemokines and the cognate chemokines receptors present in the cancers. The altered lymphocytes of the invention can be targeted to, for example, tumors listed in Table 3, by ectopic expression of the listed cognate chemokine receptor. It is to be understood that the methods described herein are not limited to the tumors and chemokine receptors listed in Table 3.

Other cell proliferative disorders may also be treated, including precancerous conditions; hematologic disorders; and immune disorders, such as autoimmune disorders including, without limitation, Addison's disease, celiac disease, diabetes mellitus type 1, Grave's disease, Hashimoto's disease, inflammatory bowel disease, multiple sclerosis, psoriasis, rheumatoid arthritis, scleroderma, and systemic lupus erythematosus

**Table 3: List of exemplary chemokines and their cognate chemokine receptors present in tumors**

| **Tumor** | **Chemokine** | **Chemokine receptor(s)** |
|---|---|---|
| Prostate tumor | CCL2 | CCR2 |
| Prostate tumor | CCL5 | CCR1, CCR3, CCR4, CCR5, DARC |
| Prostate tumor | CXCL12 | CXCR4 |
| Hodgkin lymphoma | TARC/CCL17 | CCR4, DARC |
| Melanoma | CCL2 | CCR2 |
| Melanoma | CCL5 | CCR1, CCR3, CCR4, CCR5, DARC |

Lymphocytes that can be modified by the present invention include CAR-T cells. Exemplary CAR-T cells that can be modified to express an endogenous cell surface receptor are disclosed in Table 4. In Table 4, the left column describes the cellular target of the CAR-T cell and the right column describes the scFv or the antigen binding portion of the chimeric antigen receptor expressed by the CAR-T cell.

**Table 4: List of exemplary cellular targets and CAR scFv targeting the cellular targets**

| **Cellular target** | **CAR scFv/binding portion** |
|---|---|
| CD19 | anti-CD19 |
| CD20 | anti-CD20 |
| CD22 | anti-CD22 |
| CD30 | anti-CD30 |
| CD33 | anti-CD33 |
| CD138 | anti-CD138 |
| CD171 | anti-CD171 |
| CEA | anti-CEA |
| CD123 | anti-CD123 |
| IL13 receptor alpha | IL13 |
| EGF receptor | anti-epidermal growth factor receptor |
| EGFRvIII | anti-EGFRvIII |
| ErbB | anti-ErbB |
| FAP | anti-FAP |
| GD2 | anti-GD2 |
| Glypican 3 | anti-glypican 3 |
| Her2 | anti-Her2 |
| Mesothelin | anti-mesothelin |
| ULBP and MICA/B proteins | NKG2D |
| PD1 | anti-PD1 |
| MUC1 | anti-MUC1 |
| VEGF2 | anti-VEGF2 |
| ROR1 | anti-ROR1 |

In all of the embodiments described herein, the various components for use in the methods can be produced by synthesis, or for example, using expression cassettes encoding the site-directed protein, the polynucleotide binding domain and the NATNA. The various components can be produced recombinantly in a host cell. These components can be present on a single cassette or multiple cassettes, in the same or different constructs. Expression cassettes typically comprise regulatory sequences that are involved in one or more of the following: regulation of transcription, post-transcriptional regulation, and regulation of translation. Expression cassettes can be introduced into a wide variety of organisms including bacterial cells, yeast cells, plant cells, and mammalian cells. Expression cassettes typically comprise functional regulatory sequences corresponding to the organism(s) into which they are being introduced.

In one aspect, all or a portion of the various components for use herein are produced from vectors, including expression vectors, comprising polynucleotides encoding therefor. Vectors useful for producing components for use in the present methods include plasmids, viruses (including phage), and integratable DNA fragments (*i.e.,* fragments integratable into the host genome by homologous recombination). A vector replicates and functions independently of the host genome, or can in some instances, integrate into the genome itself. Suitable replicating vectors will contain a replicon and control sequences derived from species compatible with the intended expression host cell. Transformed host cells are cells that have been transformed or transfected with the vectors constructed using recombinant DNA techniques

General methods for construction of expression vectors are known in the art. Expression vectors for most host cells are commercially available. There are several commercial software products designed to facilitate selection of appropriate vectors and construction thereof, such as insect cell vectors for insect cell transformation and gene expression in insect cells, bacterial plasmids for bacterial transformation and gene expression in bacterial cells, yeast plasmids for cell transformation and gene expression in yeast and other fungi, mammalian vectors for mammalian cell transformation and gene expression in mammalian cells or mammals, viral vectors (including retroviral, lentiviral, and adenoviral vectors) for cell transformation, and gene expression and methods to easily enable cloning of such polynucleotides. SnapGene^{™} (GSL Biotech LLC, Chicago, Ill.; snapgene.com/resources/plasmid _files/your_time_is_valuable/), for example, provides an extensive list of vectors, individual vector sequences, and vector maps, as well as commercial sources for many of the vectors.

Expression cassettes typically comprise regulatory sequences that are involved in one or more of the following: regulation of transcription, post-transcriptional regulation, and regulation of translation. Expression cassettes can be introduced into a wide variety of organisms including bacterial cells, yeast cells, mammalian cells, and plant cells. Expression cassettes typically comprise functional regulatory sequences corresponding to the host cells or organism(s) into which they are being introduced. Expression vectors can also include polynucleotides encoding protein tags (*e*.g., poly-His tags, hemagglutinin tags, fluorescent protein tags, bioluminescent tags, nuclear localization tags). The coding sequences for such protein tags can be fused to the coding sequences or can be included in an expression cassette.

In some embodiments, polynucleotides encoding one or more of the various components are operably linked to an inducible promoter, a repressible promoter, or a constitutive promoter.

Several expression vectors have been designed for expressing NATNAs, such as guide polynucleotides. *See, e.g.,* Shen, B. et al. "Efficient genome modification by CRISPR-Cas9 nickase with minimal off-target effects" (2014) Mar 2. doi: 10.1038/nmeth.2857. 10.103 8/nmeth.2857. Additionally, vectors and expression systems are commercially available, such as from New England Biolabs (Ipswich, MA) and Clontech Laboratories (Mountain View, CA). Vectors can be designed to simultaneously express a target-specific NATNA using a U2 or U6 promoter, a Cas protein and/or a dCas protein, and, if desired, a marker protein, for monitoring transfection efficiency and/or for further enriching/isolating transfected cells by flow cytometry.

Vectors can be designed for expression of various components of the described methods in prokaryotic or eukaryotic cells. Alternatively, transcription can be *in vitro,* for example using T7 promoter regulatory sequences and T7 polymerase. Other RNA polymerase and promoter sequences can be used.

Vectors can be introduced into and propagated in a prokaryote. Prokaryotic vectors are well known in the art. Typically, a prokaryotic vector comprises an origin of replication suitable for the target host cell (*e.g.,* oriC derived from *Escherichia coli,* pUC derived from pBR322, pSC101 derived from *Salmonella,* 15A origin derived from p15A, and bacterial artificial chromosomes). Vectors can include a selectable marker (*e*.*g*., genes encoding resistance for ampicillin, chloramphenicol, gentamicin, and kanamycin). Zeocin^{™} (Life Technologies, Grand Island, NY) can be used as a selection in bacteria, fungi (including yeast), plants, and mammalian cell lines. Accordingly, vectors can be designed that carry only one drug resistance gene for Zeocin for selection work in a number of organisms. Useful promoters are known for expression of proteins in prokaryotes, for example, T5, T7, Rhamnose (inducible), Arabinose (inducible), and PhoA (inducible). Furthermore, T7 promoters are widely used in vectors that also encode the T7 RNA polymerase. Prokaryotic vectors can also include ribosome binding sites of varying strength, and secretion signals (*e*.*g***.**, mal, sec, tat, ompC, and pelB). In addition, vectors can comprise RNA polymerase promoters for the expression of NATNAs. Prokaryotic RNA polymerase transcription termination sequences are also well known (*e.g*., transcription termination sequences from *Streptococcus pyogenes*).

Integrating vectors for stable transformation of prokaryotes are also known in the art (*see, e.g.,* Heap et al., Nucleic Acids Res. (2012) 40:e59).

Expression of proteins in prokaryotes is typically carried out in *E*. *coli* with vectors containing constitutive or inducible promoters directing the expression of either fusion or non-fusion proteins.

A wide variety of RNA polymerase promoters suitable for expression of the various components are available in prokaryotes (*see, e.g.,* Jiang, et al., Environ Microbiol. (2015) 81:2506-2514); Estrem et al., Genes Dev. (1999) 13:2134-2147).

In some embodiments, a vector is a yeast expression vector comprising one or more components of the above-described methods. Examples of vectors for expression in *Saccharomyces cerevisiae* include, but are not limited to, the following: pYepSec1, pMFa, pJRY88, pYES2, and picZ. Methods for gene expression in yeast cells are known in the art (*see,* e.g., Methods in Enzymology, Volume 194, "Guide to Yeast Genetics and Molecular and Cell Biology, Part A," (2004) Christine Guthrie and Gerald R. Fink (eds.), Elsevier Academic Press, San Diego, CA). Typically, expression of protein-encoding genes in yeast requires a promoter operably linked to a coding region of interest plus a transcriptional terminator. Various yeast promoters can be used to construct expression cassettes for expression of genes in yeast. Examples of promoters include, but are not limited to, promoters of genes encoding the following yeast proteins: alcohol dehydrogenase 1 (ADH1) or alcohol dehydrogenase 2 (ADH2), phosphoglycerate kinase (PGK), triose phosphate isomerase (TPI), glyceraldehyde-3-phosphate dehydrogenase (GAPDH; also known as TDH3, or triose phosphate dehydrogenase), galactose-1-phosphate uridyl-transferase (GAL7), UDP-galactose epimerase (GAL10), cytochrome ci (CYCl), acid phosphatase (PHOS) and glycerol-3-phosphate dehydrogenase gene (GPD1). Hybrid promoters, such as the ADH2/GAPDH, CYC1/GAL10 and the ADH2/GAPDH promoter also may be used. In *Schizosaccharomyces pombe,* suitable promoters include the thiamine-repressed nmtl promoter and the constitutive cytomegalovirus promoter in pTL2M.

Yeast RNA polymerase III promoters (*e*.*g*., promoters from 5S, U6 or RPR1 genes) as well as polymerase III termination sequences are known in the art *(see, e.g.,* yeastgenome.org; Harismendy et al., EMBO Journal (2003) 22:4738-4747).

In another aspect, the various components are incorporated into mammalian vectors for use in mammalian cells. A large number of mammalian vectors suitable for use with the systems of the present invention are commercially available (*e.g.,* from Life Technologies, Grand Island, NY; NeoBiolab, Cambridge, MA; Promega, Madison, WI; DNA2.0, Menlo Park, CA; Addgene, Cambridge, MA).

Vectors derived from mammalian viruses can also be used for expressing the various components of the present methods in mammalian cells. These include vectors derived from viruses such as adenovirus, papovirus, herpesvirus, polyomavirus, cytomegalovirus, lentivirus, retrovirus, vaccinia, and Simian Virus 40 (SV40) (*see, e.g.,* Kaufman, R. J., Molecular Biotechnology (2000) 16:151-160; Cooray et al., Methods Enzymol. (2012) 507:29-57). Regulatory sequences operably linked to the components can include activator binding sequences, enhancers, introns, polyadenylation recognition sequences such as from bovine growth hormone (BGH) or Simian Virus 40 (SV40), promoters, repressor binding sequences, stem-loop structures, translational initiation sequences, translation leader sequences, transcription termination sequences, translation termination sequences, primer binding sites, and the like. Commonly used promoters are constitutive mammalian promoters CMV, EF1a, SV40, PGK1 (mouse or human), MND, Ubc, CAG, CaMKIIa, and beta-Act, and others known in the art (*see, e.g.,* Khan, K.H., Advanced Pharmaceutical Bulletin (2013) 3:257-263). Furthermore, mammalian RNA polymerase III promoters, including H1 and U6, can be used.

In some embodiments, a recombinant mammalian expression vector is capable of preferentially directing expression of the nucleic acid in a particular cell type (*e.g.,* using tissue-specific regulatory elements to express a polynucleotide). Tissue-specific regulatory elements are known in the art and include, but are not limited to, the albumin promoter, lymphoid-specific promoters, neuron-specific promoters (*e.g.,* the neurofilament promoter), pancreas-specific promoters, mammary gland-specific promoters (*e.g.,* milk whey promoter), and in particular promoters of T cell receptors and immunoglobulins. Developmentally-regulated promoters are also encompassed, *e.g.,* the murine hox promoters and the alpha-fetoprotein promoter.

Numerous mammalian cell lines have been utilized for expression of gene products including HEK 293 (Human embryonic kidney) and CHO (Chinese hamster ovary). These cell lines can be transfected by standard methods (*e.g.,* using calcium phosphate or polyethyleneimine (PEI), or electroporation). Other typical mammalian cell lines include, but are not limited to: HeLa, U2OS, 549, HT1080, CAD, P19, NIH 3T3, L929, N2a, Human embryonic kidney 293 cells, MCF-7, Y79, SO-Rb50, Hep G2, DUKX-X11, J558L, and Baby hamster kidney (BHK) cells.

Methods of introducing polynucleotides (e.g., an expression vector), or ribonucleoprotein particles, into host cells are known in the art and are typically selected based on the kind of host cell. Such methods include, for example, viral or bacteriophage infection, transfection, conjugation, electroporation, calcium phosphate precipitation, polyethyleneimine-mediated transfection, DEAE-dextran mediated transfection, protoplast fusion, lipofection, liposome-mediated transfection, particle gun technology, direct microinjection, and nanoparticle-mediated delivery.

Once produced, the modified lymphocytes can be formulated into compositions for delivery to the subject to be treated. Compositions of the present invention include a modified human T cell and one or more pharmaceutically acceptable excipients. Exemplary excipients include, without limitation, carbohydrates, inorganic salts, antimicrobial agents, antioxidants, surfactants, buffers, acids, bases, and combinations thereof. Excipients suitable for injectable compositions include water, alcohols, polyols, glycerine, vegetable oils, phospholipids, and surfactants. A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like. The excipient can also include an inorganic salt or buffer such as citric acid, sodium chloride, potassium chloride, sodium sulfate, potassium nitrate, sodium phosphate monobasic, sodium phosphate dibasic, and combinations thereof.

A composition of the invention can also include an antimicrobial agent for preventing or deterring microbial growth. Nonlimiting examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can also be present in the composition. Antioxidants are used to prevent oxidation, thereby preventing the deterioration of the lymphocytes or other components of the preparation. Suitable antioxidants for use in the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant can be present as an excipient. Exemplary surfactants include: polysorbates, such as TWEEN 20 and TWEEN 80, and pluronics such as F68 and F88 (BASF, Mount Olive, New Jersey); sorbitan esters; lipids, such as phospholipids such as lecithin and other phosphatidylcholines, phosphatidylethanolamines (although preferably not in liposomal form), fatty acids and fatty esters; steroids, such as cholesterol; chelating agents, such as EDTA; and zinc and other such suitable cations.

Acids or bases can be present as an excipient in the composition. Nonlimiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof.

The amount of lymphocytes in the composition will vary depending on a number of factors, but will optimally be a therapeutically effective dose when the composition is in a unit dosage form or container (*e.g.,* a vial). A therapeutically effective dose can be determined experimentally by repeated administration of increasing amounts of the composition in order to determine which amount produces a clinically desired endpoint.

The amount of any individual excipient in the composition will vary depending on the nature and function of the excipient and particular needs of the composition. Typically, the optimal amount of any individual excipient is determined through routine experimentation, *i.e.,* by preparing compositions containing varying amounts of the excipient (ranging from low to high), examining the stability and other parameters, and then determining the range at which optimal performance is attained with no significant adverse effects. Generally, however, the excipient(s) will be present in the composition in an amount of about 1% to about 99% by weight, preferably from about 5% to about 98% by weight, more preferably from about 15 to about 95% by weight of the excipient, with concentrations less than 30% by weight most preferred. These foregoing pharmaceutical excipients along with other excipients are described in "Remington: The Science & Practice of Pharmacy," Current edition, Williams & Williams, the "Physician's Desk Reference," Current edition, Medical Economics, Montvale, NJ, and Kibbe, A.H., Handbook of Pharmaceutical Excipients, Current edition, American Pharmaceutical Association, Washington, D.C.

The compositions encompass all types of formulations and in particular those that are suited for injection, e.g., powders or lyophilates that can be reconstituted with a solvent prior to use, as well as ready for injection solutions or suspensions, dry insoluble compositions for combination with a vehicle prior to use, and emulsions and liquid concentrates for dilution prior to administration. Examples of suitable diluents for reconstituting solid compositions prior to injection include bacteriostatic water for injection, dextrose 5% in water, phosphate buffered saline, Ringer's solution, saline, sterile water, deionized water, and combinations thereof. With respect to liquid pharmaceutical compositions, solutions and suspensions will find use herein.

The pharmaceutical preparations can also be housed in a syringe, an implantation device, or the like, depending upon the intended mode of delivery and use. Preferably, the amount of the composition present is appropriate for a single dose, in a premeasured or prepackaged form.

The compositions herein may optionally include one or more additional agents, such as other medications used to treat a subject for the cancer in question or to treat known side-effects from the treatment. For example, T cells release cytokines into the bloodstream, which can lead to dangerously high fevers and precipitous drops in blood pressure. This condition is known as cytokine release syndrome (CRS). In many patients, CRS can be managed with standard supportive therapies, including steroids and immunotherapies, such as tocilizumab (Actemra^{™}, Genetech, South San Francisco, CA) that block IL-6 activity.

The invention further provides for a composition comprising a modified human T cell as described and a pharmaceutically acceptable excipient for use in the treatment of cancer comprising administering a therapeutically effective amount of said composition to a human subject in need thereof. At least one therapeutically effective cycle of treatment with a modified lymphocyte composition will be administered to a subject. By "therapeutically effective cycle of treatment" is intended a cycle of treatment that, when administered, brings about a positive therapeutic response with respect to treatment of an individual for the disease in question. By "positive therapeutic response" is intended that the individual undergoing treatment according to the invention exhibits an improvement in one or more symptoms of the disease, including such improvements as tumor reduction and/or reduced need for lymphocyte therapy.

In certain embodiments, multiple therapeutically effective doses of compositions comprising the lymphocytes or other medications will be administered. The compositions of the present invention are typically, although not necessarily, administered via injection, such as subcutaneously, intradermally, intravenously, intraarterially, intramuscularly, intraperitoneally, intramedullary, intratumorally, intranodally), by infusion, or locally. The pharmaceutical preparation can be in the form of a liquid solution or suspension immediately prior to administration. The foregoing is meant to be exemplary as additional modes of administration are also contemplated. The pharmaceutical compositions may be administered using the same or different routes of administration in accordance with any medically acceptable method known in the art.

The actual dose to be administered will vary depending upon the age, weight, and general condition of the subject as well as the severity of the condition being treated, the judgment of the health care professional, and particular lymphocytes being administered. Therapeutically effective amounts can be determined by those skilled in the art, and will be adjusted to the particular requirements of each particular case.

Generally, a therapeutically effective amount of lymphocytes will range from a total of about 1 × 10⁵ to about 1 × 10¹⁰ lymphocytes or more per patient, such as 1 × 10⁶ to about 1 × 10¹⁰, *e.g.,* 1 × 10⁷ to 1 × 10⁹, such as 5 × 10⁷ to 5 × 10⁸, or any amount within these ranges. Other dosage ranges can be 1×10⁴ to 1×10¹⁰ cells per kg/bodyweight. The total number of lymphocytes can be administered in a single bolus dose, or can be administered in two or more doses, such as one or more days apart. The amount of compound administered will depend on the potency of the specific lymphocyte composition, the disease being treated and the route of administration.

Additionally, the doses can comprise a mixture of lymphocytes, such as a mix of CD8+ and CD4+ cells. If a mix of CD8+ and CD4+ cells is provided, the ratio of CD8+ to CD4+ cells can be for example, 1:1, 1:2 or 2:1, 1:3 or 3:1, 1:4 or 4:1, 1:5 or 5:1, etc.

The modified lymphocytes can be administered prior to, concurrent with, or subsequent to other agents. If provided at the same time as other agents, the modified lymphocytes can be provided in the same or in a different composition. Thus, the lymphocytes and other agents can be presented to the individual by way of concurrent therapy. By "concurrent therapy" is intended administration to a subject such that the therapeutic effect of the combination of the substances is caused in the subject undergoing therapy. For example, concurrent therapy may be achieved by administering a dose of a pharmaceutical composition comprising modified lymphocytes and a dose of a pharmaceutical composition comprising at least one other agent, such as another chemotherapeutic agent, which in combination comprises a therapeutically effective dose, according to a particular dosing regimen. Similarly, modified lymphocytes and therapeutic agents can be administered in at least one therapeutic dose. Administration of the separate pharmaceutical compositions can be performed simultaneously or at different times (e.g., sequentially, in either order, on the same day, or on different days), as long as the therapeutic effect of the combination of these substances is caused in the subject undergoing therapy.

### EXPERIMENTAL

Aspects of the present invention are further illustrated in the following Examples. Efforts have been made to ensure accuracy with respect to numbers used (*e*.*g*., amounts, concentrations, percent changes, etc.) but some experimental errors and deviations should be accounted for. Unless indicated otherwise, temperature is in degrees Centigrade and pressure is at or near atmospheric. It should be understood that these Examples, while indicating some embodiments of the invention, are given by way of illustration only.

The following Examples are not intended to limit the scope of what the inventors regard as various aspects of the present invention.

### Example 1: Preparation of cytotoxic T cells (CD4+ and CD8+) from peripheral blood mononuclear cells (PBMCs)

This Example illustrates the preparation of CD4+ and CD8+ T cells from donor PBMCs. Not all of the following steps are required nor must the order of the steps be as presented.

### T cell isolation, activation, and expansion from fresh PBMCs

CD4+ and CD8+ T cells were prepared from donor PBMCs. Approximately 350 million donor PBMCs were obtained from a commercial supplier (AllCells, Alameda, CA). The PBMCs were small aliquots from an apheresis leuko pak. The leuko pak was collected using the Spectra Optia^{®} Apheresis System (Terumo BCT, Inc., Lakewood, CO) (approximately 90% of these cells were mononuclear cells (MNCs). These fresh MNC products were processed to deplete red blood cells using an ammonium chloride solution and were processed to deplete platelets by centrifugation.

PBMCs were enumerated using acetic acid with methylene blue using the Countess II FL Automated Cell Counter (ThermoFisher Scientific, Waltham, MA). A 1:10 dilution in acetic acid with methylene blue was sufficient for an accurate count. Cells were then centrifuged at 300 x g for approximately 7-10 minutes to pellet cells. The supernatant was discarded and the cell pellet gently resuspended in 1 mL Complete RPMI (Gibco 61870-036, ThermoFisher Scientific, Waltham, MA) 500 ml RPMI + 10% H.I. FBS (Corning 35-011-CV, Corning, NY) + 5.5 ml 100X Antibiotic-Antimycotic (Gibco 15240-062, ThermoFisher Scientific, Waltham, MA) at 37°C with 100 µL DNaseI (Stemcell Technologies, Cambridge, MA). Then 29 mL warm Complete RPMI at 37°C was added.

The cells were split into 3-T175 Tissue Culture (Falcon 353112, Corning, NY) treated flasks with a minimum volume of 20 mL/flask using Complete RPMI. Flasks were then placed into 37°C, 5% CO2 incubator for approximately 4 hours. After the 4-hour incubation period, flasks were gently removed from the incubator and the suspension cells (non-adherent cells) were pipetted into 50 mL conical tubes. Cells that had adhered to the flask were monocytes/macrophages and were discarded. The cells in suspension contained the lymphocytes (NK, B, and T cells).

Cells were centrifuged at 300 x g for approximately 7-10 minutes to pellet cells. The supernatant was discarded and the cell pellet gently resuspended in an appropriate volume of Complete RPMI and enumerated using the Countess II FL Automated Cell Counter (ThermoFisher Scientific, Waltham, MA). Cells were resuspended at 1e6 cells/mL in Complete RPMI + 40 U/mL rhIL-2 (R&D Systems 202-IL-050, Minneapolis, MN (50 µg of rhIL-2 was reconstituted at 50 µg/mL using sterile 100 mM acetic acid + 0.1% BSA. 50 µg/mL is equivalent to 200,000 U/mL using an ED50 of 0.25 ng/mL) and plated into washed, coated T-175 flask(s).

One day prior to the delivery of PBMCs, 2-T175 tissue culture treated flasks were coated with anti-CD3 (HIT3A, ThermoFisher Scientific, Waltham, MA) at 1 µg/mL and anti-CD28 (BD Biosciences, San Jose, CA) at 5 µg/mL in PBS. Flask tops were wrapped with parafilm and then the coated flasks were placed at 4°C overnight. For one T175 flask, 20 mL of PBS + 20 µL - 1 mg/mL anti-CD3 + 100 µL - 1 mg/mL anti-CD28 was added. The T175 flasks that were coated the previous day with anti-CD3 and anti-CD28 were removed from 4°C and washed once with PBS. The maximum volume for T-175 flask was 100 - 200 mL. If total volume was greater than 200 mL, the volume was split equally into the appropriate number of T-175 flasks. For example, for a total volume of 250 mL, 125 mL was plated into 2 coated T-175 flasks. Flasks were placed in a 37°C, 5% CO2 incubator for 3 days to activate T cells. On the morning of the third day, T cells were transferred to 50 mL conical tubes and centrifuged at 300 x g for approximately 7-10 minutes to pellet cells. The supernatant was discarded and the pellet was gently resuspended and the T cells pooled in an appropriate volume of Complete RPMI and enumerated. The enumerated T cells were resuspended at 1×10⁶cells/mL in Complete RPMI + 40 U/mL rhIL-2 and plated into as many T-175 suspension flask as required (maximum volume per flask was 250 mL). Flasks were placed in 37°C, 5% CO2 incubator for approximately 24 hours before use. After 24 hours (t=1), T cells were enumerated again and used for transfections and other functional assays. The purity of CD3+CD4+ and CD3+CD8+ T cells can be assessed by fluorescence activated flow cytometry (FACS) as described in Example 4.

Following the guidance of the present Specification and Examples, other types of lymphocytes can be isolated, activated and expanded from fresh PBMCs by one of ordinary skill in the art.

### Example 2: Cloning, expression, production, and assembly of NATNA/dCas9-VP64 ribonucleoproteins (RNPs)

This Example describes a method for cloning, expressing, and producing NATNA/dCas9-VP64 complexes using an *E*. *coli* expression system and an RNA *in vitro* transcription system. Not all of the following steps are required nor must the order of the steps be as presented.

### Cloning of dCas9-VP64

The *S. pyogenes* (Spy) dCas9-VP64 sequence that codes for a catalytically inactive Cas9 (dCas9) (SEQ ID NO: 19) fused to a VP64 (SEQ ID NO: 18) activator cassette is codon optimized for expression in *E*. *coli* cells. At the C-terminus, two nuclear localization sequences (NLS) (SEQ ID NO: 20) are added, and the coding sequence is inserted into a vector adjacent to a suitable bacterial promoter such as the T7 promoter. Oligonucleotide sequences coding for dCas9-VP64 can be provided to commercial manufacturers for synthesis (*e*.*g*., Integrated DNA Technologies, Coralville, IA). DNA sequences are then cloned into suitable bacterial expression vectors with standard cloning methods.

### Expression and purification of dCas9-VP64 protein

The dCas9-VP64 protein (*e*.*g*. SpydCas9 protein) is expressed from a bacterial expression vector in *E*. *coli* (such as BL21 (DE3)) and can be purified using affinity chromatography, ion exchange, and size exclusion chromatography according to methods described in Jinek, et al., Science (2012) 337:816-821.

### Production of NATNA components

The production of NATNA components has been described herein and elsewhere. NATNA components are produced by *in vitro* transcription (*e*.*g*., T7 Quick High Yield RNA Synthesis Kit, New England Biolabs, Ipswich, MA) from double-stranded DNA templates incorporating a T7 promoter at the 5' end of the DNA sequences, or the sequences are provided to commercial manufacturers for synthesis (*e*.*g*., Integrated DNA Technologies, Coralville, IA).

The double-stranded DNA templates for the specific NATNA components used in the Examples are assembled by PCR using 3' overlapping primers containing the corresponding DNA sequences to the NATNA components. The unique oligonucleotide sequences of the overlapping primers can be designed as described in U.S. Patent Nos. 9,650,617, U.S. 9,771,601, and PCT Publication WO 2016/123230, published 4 August 2016. Exemplary crRNA sequences for CCR2 tiling are listed in Table 5.

**Table 5: Exemplary crRNA sequences for CCR2 tiling**

| **Type of RNA component** | **DNA-binding sequence** | **DNA-binding Sequence SEQ ID NOS:** | **NATNA SEQ ID NOS:** |
|---|---|---|---|
| crRNA_CCR2 | CCR2 Target 1-40 | SEQ ID NOS: 61-100 | SEQ ID NOS: 101-140 |
| TracrRNA | N/A | N/A | 141 |

The DNA primers are present at a concentration of 2 nM each. Two outer DNA primers corresponding to the T7 promoter and the 3' end of the RNA sequence are used at 640 nM to drive the amplification reaction. PCR reactions are performed using KAPA HiFi Hot Start Polymerase (Kapa Biosystems, Inc., Wilmington, MA) and can contain 0.5 units of polymerase, 1x reaction buffer, and 0.4 mM dNTP. PCR assembly reactions are carried out using the following thermal cycling conditions: 95°C for 2 minutes, 30 cycles of 20 seconds at 98°C, 20 seconds at 62°C, 20 seconds at 72°C, and a final extension at 72°C for 2 minutes. DNA quality is evaluated by agarose gel electrophoresis (1.5%, SYBR^{®} Safe, Life Technologies, Grand Island, NY).

Between 0.25-0.5mg of the DNA template for a NATNA component is transcribed using T7 Quick High Yield RNA Synthesis Kit (New England Biolabs, Ipswich, MA) for approximately 16 hours at 37°C. The transcription reaction is DNAse I treated (New England Biolabs, Ipswich, MA) and can be purified using the GeneJet RNA cleanup and concentration kit (Life Technologies, Grand Island, NY). NATNA yield is quantified using the Nanodrop^{™} 2000 system (Thermo Scientific, Wilmington DE). The quality of the transcribed RNA is checked by agarose gel electrophoresis (2%, SYBR^{®} Safe, Life Technologies, Grand Island, NY).

### Assembly of NATNA/dCas9-VP64 RNPs

*S. pyogenes* dCas9-VP64 is tagged at the C-terminus with two nuclear localization sequences (NLS) and is recombinantly expressed in *E*. *coli* and purified using chromatographic methods. Ribonucleoprotein complexes are formed at a concentration of 40 pmol dCas9-VP64 protein: 120 pm NATNA. Prior to assembly with dCas9-VP64, each of the crRNA NATNAs (SEQ ID NOS: 101-140) and the tracrRNA NATNA (SEQ ID NO: 141) is diluted to the desired total concentration (120 pmol) in a final volume of 2 µl, incubated for 2 minutes at 95°C, removed from a thermocycler, and allowed to equilibrate to room temperature. The dCas9-VP64 protein is diluted to an appropriate concentration in binding buffer (20 mM HEPES, 100 mM KCl, 5 mM MgCl₂, 1 mM DTT, and 5% glycerol at pH 7.4) to a final volume of 3 µl and mixed with the 2 µl of the crRNA NATNA/tracrRNA NATNA followed by incubation at 37°C for 30 minutes.

Following the guidance of the present Specification and Examples, other types of transcription activation protein complexes can be prepared by one of ordinary skill in the art.

### Example 3: Nucleofection of T cells (CD4+ and CD8+) from PBMCs with NATNA/dCas9-VP64 RNPs

This Example describes the nucleofection of activated T cells with NATNA/dCas9-VP64 RNPs. Not all of the following steps are required nor must the order of the steps be as presented.

### Cell transfections using NATNA/dCas9-VP64 RNPs

The NATNA/dCas9-VP64 RNPs of Example 2 are transfected into primary activated T cells (CD4+ and CD8+) (from Example 1) using the Nucleofector^{™} 96-well Shuttle System (Lonza, Allendale, NJ). The NATNA/dCas9-VP64 RNPs are dispensed in a 5 µl final volume into individual wells of a 96-well plate. Complete RPMI 1640 is added at 150 µL/well to a TC 96 well plate for suspension cells. The suspended T cells are pelleted by centrifugation for 10 minutes at 200 × g, and cells are washed with calcium and magnesium-free phosphate buffered saline (PBS)/0.5% BSA. Cells are pelleted by centrifugation for 3 minutes at 200 × g, the PBS/BSA aspirated, and the cell pellet re-suspended in 10 mL of calcium and magnesium-free PBSBSA. The cells are counted using the Countess^{®} II Automated Cell Counter (Life Technologies; Grand Island, NY).

2.2 × 10⁷ cells are transferred to a 1.5 ml microfuge tube and pelleted. The PBSBSA is aspirated and the cells re-suspended in Nucleofector^{™} P4 (Lonza, Allendale, NJ) solution to a density of 1 × 10⁷ cells/ml per sample. 20 µl of the cell suspension is then added to each individual well containing 5 µl of ribonucleoprotein complexes, and the entire volume from each well is transferred to a well of a 96-well Nucleocuvette^{™} Plate (Lonza, Allendale, NJ). The plate is loaded onto the Nucleofector^{™} 96-well Shuttle^{™} (Lonza, Allendale, NJ) and cells nucleofected using the 96CA137 Nucleofector^{™} program (Lonza, Allendale, NJ). Post-nucleofection, 80 µl complete RPMI 1640 + 40 U/ml rhIL-2 (RPMI 1640; Gibco 61870-036, 500 ml RPMI + 10% H.I. FBS + 5.5 ml 100X Antibiotic-Antimycotic) is added to each well, and 100 µl of the cell suspension is transferred to a 96-well cell culture plate containing 100 µl prewarmed RPMI 1640 complete culture medium. The plate is transferred to a tissue culture incubator and maintained at 37°C in 5% CO₂ for 48 hours.

Following the guidance of the present Specification and Examples, other types of lymphocytes, such as CAR-T cells, engineered TCR T cells, or NK cells, can be transduced with transcription activation protein complexes by one of ordinary skill in the art.

### Example 4: FACS assay to detect cell surface expression of CCR2 on activated primary T cells

This Example describes the determination of the status of CCR2 expression on activated primary T cells with FACS analysis using CCR2-specific antibodies. Not all of the following steps are required nor must the order of the steps be as presented.

### Cell staining

Activated primary T cells are screened for endogenous or activated CCR2 cell surface expression using FACS analysis and CCR2-specific antibodies. 1 × 10⁵ cells per sample of activated primary T cells (Example 1) are centrifuged in at 200 × g for 5 minutes to pellet cells. The supernatant is decanted. Then 100 µl of FACS buffer (1 × PBS with 2% FBS) is added to the cell pellet.

Anti-CCR2 antibody, such as Anti-CCR2 antibody (monoclonal mouse antibody clone 7A7) at 0.4 mg/ml (ab176390, Abcam, Cambridge, MA), is added at a dilution of 1:50 and the samples are incubated on ice for 30 minutes. The samples are washed three times by adding 200 µl of FACS staining buffer, and centrifuging at approximately 200 × g for 5 minutes. Then, a Goat Anti-Mouse IgG H&L labelled with Alexa Fluor^{™} 647 (ab15011, Abcam, Cambridge, MA) at 2 mg/ml is added at a dilution of 1:2000 and the samples are incubated on ice for 30 minutes. The cells are washed three times by adding 200 µl of FACS staining buffer, and centrifuging at 200 × g for 5 minutes. The cells are stained with anti-CD3 antibody such as Brilliant Violet 421^{™} anti-human CD3 Antibody (no 317343, BioLegend San Diego, CA) following the manufacturer's protocol. After the washes, cells are resuspended in 100 µl FACS buffer and kept on ice or are ready for analysis.

Isotype controls and native Cas9RNP controls are similarly stained for reference. Stained cells are then sorted on a LSR II flow cytometer (BD laboratories, San Jose, CA) and the population of FITC positive fluorescent cells tallied.

### FACS assay

Upregulation of CCR2 expression is measured by an increase in detected fluorescence of a NATNA/dCas9-VP64-nucleofected sample compared to the measured fluorescence of a non-transfected control. Increase in fluorescence from the flow cytometer is used to demonstrate that NATNA/dCas9-VP64 can upregulate transcription of said gene target.

Following the guidance of the present Specification and Examples, endogenous cell surface expression of other chemokine receptors can be determined on other types of lymphocytes, such as CAR-T cells, engineered TCR T cells, or NK cells, by one of ordinary skill in the art.

### Example 5: Screening for guide target sites in the CCR2 transcriptional start site with NATNA/dCas9-VP64 RNPs in primary T cells

This Example describes the tiling of the genomic locus for the transcriptional start site of CCR2 in activated T cells with NATNA/dCas9-VP64 RNPs for transcriptional activation. This method is adapted from Gilbert et al., Cell (2013) 154:442-451; Gilbert etal., Cell (2014) 159:647-661; and Simeonov et al., Nature (2017) 549:111-115. Transcriptional start sites (TSS) are predicted for CCR2 in *the Homo sapiens* chromosome 3, GRCh38.p7 Primary Assembly as described in Abugessaisa et al., Scientific Data (2017) 4:170107and using the NCBI server (ncbi.nlm.nih.gov). Not all of the following steps are required nor must the order of the steps be as presented. The genomic region for the CCR2 transcriptional start site is predicted using the NCBI server. Transcriptional start sites predicted for CCR2 are shown in Table 6.

**Table 6: Transcriptional start sites predicted for CCR2, coordinates are in Homo sapiens chromosome 3, GRCh38.p7 primary assembly**

| **Gene** | **Gene locus** | **Transcriptional start site** |
|---|---|---|
| CCR2 | NC_000003.12 (46353744..46360940) | 46,353,734 |
| | | 46,353,811 |
| | | 46,353,860 |
| | | 46,353,878 |
| | | 46,354,021 |
| | | 46,354,082 |
| | | 46,354,126 |
| | | 46,354,142 |

The genomic region for the CCR2 transcriptional start site is screened with NATNA/dCas9-VP64 for optimized transcriptional activation by staining and detecting cell surface expression of CCR2 and comparing the cell surface expression to untreated T cells. The transcriptional start sites (TSS) for CCR2 are identified in Table 7. Then RNA guides are designed by identifying all PAM sequences (e.g., NGG) 300 bp upstream and downstream of the TSS and selection of one or more 20 nucleotide sequences (sesPN(s), e.g., sesRNA(s) and/or sesDNA(s)) that is/are 5' adjacent to PAM sequences.

**Table 7: Exemplary PAMs and sesPN sequences 300 bp upstream and downstream the chr3:46,353,734 transcriptional start site**

| **SEQ ID NO:** | **PAM locus** | **sesPN location** | **sesPN with NGG PAM sequence** |
|---|---|---|---|
| SEQ ID NO: 21 | chr3.46353656- | chr3:46353656-46353678 | GGTATCATGGATTCAGAATCTGG |
| SEQ ID NO: 22 | chr3.46353669- | chr3:46353669-46353691 | TGCTGGTTTCAGTGGTATCATGG |
| SEQ ID NO: 23 | chr3.46353677- | chr3:46353677-46353699 | ATCATGTGTGCTGGTTTCAGTGG |
| SEQ ID NO: 24 | chr3.46353686- | chr3:46353686-46353708 | TTTACTGCGATCATGTGTGCTGG |
| SEQ ID NO: 25 | chr3.46353710- | chr3:46353710-46353732 | ATAGTGGAGGAAGTATAATGAGG |
| SEQ ID NO: 26 | chr3.46353723- | chr3:46353723-46353745 | AAGGGTATTGGTGATAGTGGAGG |
| SEQ ID NO: 27 | chr3.46353726- | chr3:46353726-46353748 | ATAAAGGGTATTGGTGATAGTGG |
| SEQ ID NO: 28 | chr3.46353733+ | chr3:46353733-46353755 | CACCAATACCCTTTATTCTCTGG |
| SEQ ID NO: 29 | chr3.46353735- | chr3:46353735-46353757 | TTCCAGAGAATAAAGGGTATTGG |
| SEQ ID NO: 30 | chr3.46353741- | chr3:46353741-46353763 | TTCATGTTCCAGAGAATAAAGGG |
| SEQ ID NO: 31 | chr3.46353742- | chr3:46353742-46353764 | TTTCATGTTCCAGAGAATAAAGG |
| SEQ ID NO: 32 | chr3.46353783+ | chr3:46353783-46353805 | TCATGCAAATTATCACTAGTAGG |
| SEQ ID NO: 33 | chr3.46353797+ | chr3:46353797-46353819 | ACTAGTAGGAGAGCAGAGAGTGG |
| SEQ ID NO: 34 | chr3.46353809+ | chr3:46353809-46353831 | GCAGAGAGTGGAAATGTTCCAGG |
| SEQ ID NO: 35 | chr3.46353827- | chr3:46353827-46353849 | ATCTTGTGGGTCTTTATACCTGG |
| SEQ ID NO: 36 | chr3.46353840- | chr3:46353840-46353862 | TCTGAGCTTCTTTATCTTGTGGG |
| SEQ ID NO: 37 | chr3.46353841- | chr3:46353841-46353863 | CTCTGAGCTTCTTTATCTTGTGG |
| SEQ ID NO: 38 | chr3.46353855+ | chr3:46353855-46353877 | AGCTCAGAGTCGTTAGAAACAGG |
| SEQ ID NO: 39 | chr3.46353869+ | chr3:46353869-46353891 | AGAAACAGGAGCAGATGTACAGG |
| SEQ ID NO: 40 | chr3.46353870+ | chr3:46353870-46353892 | GAAACAGGAGCAGATGTACAGGG |
| SEQ ID NO: 41 | chr3.46353892+ | chr3:46353892-46353914 | GTTTGCCTGACTCACACTCAAGG |
| SEQ ID NO: 42 | chr3.46353897- | chr3:46353897-46353919 | TGCAACCTTGAGTGTGAGTCAGG |
| SEQ ID NO: 43 | chr3.46353929+ | chr3:46353929-46353951 | TTTCAAAATTAATCCTATTCTGG |
| SEQ ID NO: 44 | chr3.46353942- | chr3:46353942-46353964 | TGGGTTGAGGTCTCCAGAATAGG |
| SEQ ID NO: 45 | chr3.46353955- | chr3:46353955-46353977 | GAACATTGTACATTGGGTTGAGG |
| SEQ ID NO: 46 | chr3.46353961- | chr3:46353961-46353983 | AGTCAGGAACATTGTACATTGGG |
| SEQ ID NO: 47 | chr3.46353962- | chr3:46353962-46353984 | CAGTCAGGAACATTGTACATTGG |
| SEQ ID NO: 48 | chr3.46353963+ | chr3:46353963-46353985 | CAATGTACAATGTTCCTGACTGG |
| SEQ ID NO: 49 | chr3.46353977- | chr3:46353977-46353999 | ATAGTTCTTCTTTTCCAGTCAGG |
| SEQ ID NO: 50 | chr3.46354030- | chr3:46354030-46354052 | CGGAGATACAGGGCAACTAATGG |
| SEQ ID NO: 51 | chr3.46354040- | chr3:46354040-46354062 | AAAGTGAAGGCGGAGATACAGGG |
| SEQ ID NO: 52 | chr3.46354041- | chr3:46354041-46354063 | GAAAGTGAAGGCGGAGATACAGG |
| SEQ ID NO: 53 | chr3.46354047+ | chr3:46354047-46354069 | TCTCCGCCTTCACTTTCTGCAGG |
| SEQ ID NO: 54 | chr3.46354050- | chr3:46354050-46354072 | TTTCCTGCAGAAAGTGAAGGCGG |
| SEQ ID NO: 55 | chr3.46354053- | chr3:46354053-46354075 | AAGTTTCCTGCAGAAAGTGAAGG |
| SEQ ID NO: 56 | chr3.46354081- | chr3:46354081-46354103 | GAAACTTGGCATGCAGAAGTAGG |
| SEQ ID NO: 57 | chr3.46354095- | chr3:46354095-46354117 | CAGATCTAGAGGTAGAAACTTGG |
| SEQ ID NO: 58 | chr3.46354100+ | chr3:46354100-46354122 | TTTCTACCTCTAGATCTGTTTGG |
| SEQ ID NO: 59 | chr3.46354106- | chr3:46354106-46354128 | ACTGAACCAAACAGATCTAGAGG |
| SEQ ID NO: 60 | chr3.46354130+ | chr3:46354130-46354152 | GCTGAGAAGCCTGACATACCAGG |

Selection criteria can include, but are not limited to, homology to other regions in the genome, percent G-C content, melting temperature, presence of homopolymer within the spacer, and other criteria known to one skilled in the art. NATNAs are produced as described in Example 2, or sequences are provided to commercial manufacturers for synthesis (e.g., Integrated DNA Technologies, Coralville, IA; Eurofins Genomics, Luxembourg; Synthego, Redwood City, CA; Axolabs, Kulmbach, Germany). Then individual NATNA/dCas9-VP64 RNPs for screening are prepared as in Example 2 and transfected into primary T cells as described in Example 3. The resulting upregulation in cell surface expression of CCR2 from the endogenous locus after transcriptional activation with NATNA/dCas9-VP64 RNPs is determined as described in Example 4. NATNA guides are ranked for upregulation of cell surface expression of CCR2 and the desired NATNA guides (typically, top 5) are chosen.

Following the guidance of the present Specification and Examples, other genomic loci can be tiled for transcriptional activation with transcription activation molecules by one of ordinary skill in the art.

### Example 6: Culturing CCR2 enhanced primary T cells (CD4+ and CD8+)

This Example describes the nucleofection and culturing of activated primary T cells with optimized CCR2 transcriptional activator NATNA/dCas9-VP64. Not all of the following steps are required nor must the order of the steps be as presented.

NATNA guides for transcriptional activation of the endogenous CCR2 locus gene expression are chosen from guides identified in Example 5. Activated primary T cells are nucleofected with the optimized CCR2 specific NATNA/dCas9-VP64 RNPs as described in Example 3. NATNA/dCas9-VP64 transfected and mock-transfected primary T cells are grown in suspension culture in coated T-175 flask in Complete RPMI + 40 U/mL rhIL-2 at 37°C in 5% CO₂. Cells are split every 2 days to a density of 1 × 10⁶ cells/ml. Cells are pelleted at 200 × g for 5 minutes. 1 ml of Complete RPMI is added to the cell pellet and the cells are enumerated as described above using a Countess II FL Automated Cell Counter (ThermoFisher Scientific, Waltham, MA). Cells are diluted to 1 × 10⁶ cells/ml and transferred to coated T-175 flask in Complete RPMI + 40 U/mL rhIL-2 at 37°C in 5% CO₂. The fraction of CD3+CD4+CCR2+ and CD3+CD8+ CCR2+ T cells are assessed by FACS as described in Example 4.

Following the guidance of the present Specification and Examples, other enhanced lymphocytes can be transfected and propagated in tissue culture by one of ordinary skill in the art.

### Example 7: qPCR to detect CCR2 expression levels in activated primary T cells

This Example describes the determination of CCR2 expression levels in activated primary T cells using qPCR. Not all of the following steps are required nor must the order of the steps be as presented.

NATNA/dCas9-VP64-transfected and mock-transfected primary T cells are grown in 96 well tissue culture plates in Complete RPMI + 40 U/mL rhIL-2 at 37°C in 5% CO₂. Cells are harvested by centrifuging at 200 × g for 5 minutes to pellet cells. Pelleted cells are washed one time with 200 µl ice cold PBS. Cells are enumerated as described above using a Countess II FL Automated Cell Counter (ThermoFisher Scientific, Waltham, MA). Total RNA is extracted from 1 × 10⁵ cells using RNeasy Micro Plus Kit (QIAGEN, Hilden, Germany) following the manufacturer's protocol. The RNA is quantified using a Nanodrop 8000^{™} (Thermo Scientific, Waltham, MA). After quantification, RNA is reverse-transcribed into cDNA using High Capacity cDNA Reverse Transcription kit^{™} (Applied Biosystems, Thermo Scientific, Waltham, MA). Within each experiment the same amount of cDNA is used, in the range 14-60 ng/reaction. Real-time qPCR is performed using TaqMan gene expression assays (Thermo Scientific, Waltham, MA) following the manufacturer's protocol. The whole genome sequence is provided to a manufacturer such as Applied Biosystems (ThermoScientific, Waltham, MA) for qPCR primer optimization and manufacturing. qPCR is performed following the manufacturer's protocol on a qPCR machine such as the Applied Biosystems StepOnePlus^{™} (Thermo Scientific, Waltham, MA). Results of baseline and ectopic expression of CCR2 are analyzed using the software suite accompanying the Applied Biosystems StepOnePlus qPCR machine.

Following the guidance of the present Specification and Examples, the expression of gene products of other genomic loci can be determined by one of ordinary skill in the art.

### Example 8: Time course of CCR2 expression on activated primary T cells

This Example describes the determination of a time course of CCR2 ectopic expression levels on activated primary T cells after transcriptional activation with CCR2 specific NATNA/dCas9-VP64 using qPCR and FACS. Not all of the following steps are required nor must the order of the steps be as presented.

NATNA/dCas9-VP64-transfected and mock-transfected primary T cells are grown as described in Example 6. Cells are harvested by centrifuging at 200 × g for 5 minutes to pellet cells. Pelleted cells are washed one time with 1 ml ice cold PBS. Cells are enumerated as described above using a Countess II FL Automated Cell Counter^{™} (ThermoFisher Scientific, Waltham, MA). 1 × 10⁵ cells are used per sample. CCR2 cell surface expression and the fraction of CCR2 expression cells are detected as described in Example 4 by FACS. In accordance with Example 7, mRNA levels of CCR2 are determined using qPCR. Cell samples are taken at 24 hours, 48 hours, 72 hours, 5 days, and 10 days after transfection and CCR2 expression is tracked using the above described methods.

Following the guidance of the present Specification and Examples, the temporal expression of ectopically transcriptional activated gene products of other genomic loci can be determined by one of ordinary skill in the art.

### Example 9: Transwell migration assay using CCL2 chemokine and CCR2+ enhanced primary T cells

This Example describes the determination of CCL2 chemokine-mediated migration (chemotaxis) activity of primary T cells after transcriptional activation with CCR2 specific NATNA/dCas9-VP64 using an *in-vitro* transwell migration assay. Not all of the following steps are required nor must the order of the steps be as presented.

NATNA/dCas9-VP64-transfected and mock-transfected primary T cells are grown as described in Example 6. For the migration assay, co-culturing tissue culture plates such as Costar (Corning, NY) 24-well plates with 3 µm pore size are used. Cells are harvested by centrifuging at 200 × g for 5 minutes to pellet cells. Pelleted cells are washed one time with 1 ml ice cold PBS. Cells are enumerated as described above using a Countess II FL Automated Cell Counter^{™} (ThermoFisher Scientific, Waltham, MA). 2 × 10⁵ cells in complete RPMI + 40 U/mL rhIL-2 per sample is used. 100 µl of cells (2 × 10⁵ ) are placed in the upper chamber of the co-culturing tissue culture plate. The bottom chamber is filled with 500 µl of complete RPMI + 40 U/ml rhIL-2 with or without CCL2 (100 ng/ml, Recombinant Human CCL2 (MCP-1) from Biolegend, San Diego, CA). Cells are incubated at 37°C for 2 hours, 4 hours, 8 hours, and 12 hours in 5% CO₂ in the chamber to allow for migration. Migrated cells are collected from the bottom chambers and counted using FACS as described in Example 4.

Following the guidance of the present Specification and Examples, the chemotaxis activity of other lymphocytes after ectopic transcriptional activation can be analyzed by one of ordinary skill in the art.

### Example 10: Cytotoxicity assay of CCR2+ enhanced activated CAR19-T cells

This Example describes the determination of cytotoxicity or cell killing by CCR2+ CAR19-T cells after transcriptional activation with CCR2-specific NATNA/dCas9-VP64 using a CAR19 specific killing assay. Not all of the following steps are required nor must the order of the steps be as presented.

Activated CD4+ or CD8+ cells can only kill target cells that present matching peptide MHC complexes on their surface to their endogenous T cell receptor (TCR). However, since T cells rearrange the TCR during their maturation, the cognate peptide MHC complex is seldom known.

T cells are engineered for specific killing by introducing a CAR (Chimeric Antigen Receptor) protein. An anti-CD19 CAR (CAR19) is introduced into T cells to make such cells specific for CD19 positive cells as detailed below.

### Design and cloning of CAR19 into a lentivirus vector

Design and cloning of CAR receptors have been described elsewhere and the design has been adapted from Kochenderfer et al., J. Immunother. (2009) 32:689-702. The CAR contained an N terminal secretion signal (hGMCF signal peptide), an scFv portion specific for CD19, followed by a hinge region, CD28 transmembrane and effector region, and a CD3ζ effector region. The vector carrying the genomic information for the Lentivirus protein expression was obtained from Cellecta (Cellecta, Mountain View, CA). Based on the pR-CMV-Cas9-2A-Hygro-NTI backbone, an EF1-alpha promoter was cloned into the vector, replacing the CMV promoter and the sequence for the CAR receptor was introduced replacing the Cas9 sequence creating the EF1-alpha-CAR19-Lentivirus vector.

### CAR-lentivirus production

Lentivirus production has been described in detail elsewhere (*see e.g.,* Kaufman R. J., (2000) Molec. Biotech. (2000) 16: 151-160). To transduce 1.5 × 10⁷ primary T cells with lentivirus, virus produced from 40 × 10⁶ HEK293T cells were needed. HEK 293 T cells for lentivirus production were obtained from a commercial manufacturer (the American Type Culture Collection (ATCC^{®} CRL-11268^{™}). On the first day, HEK 293 T cells were plated at 4 × 10⁵ cells/ml on tissue culture coated 15 cm plates in 20 ml complete growth media (DMEM supplemented with 10% fetal bovine serum and 1 × penicillin streptomycin) and grown at 37°C, 5% CO₂ in an incubator. Cells from five 15 cm plates were used per experiment. On the second day, the cells were transfected with the EF1-CAR19-lentivirus vector for lentivirus production. Per 15 cm plate, a transfection mix of 4 µg of CAR-lentivirus vector, 40 µl of packaging mix (CPCP-K2A, Cellecta, Inc., Mountain View, CA) 42 µl Mirus TransIT^{®}-293 Transfection Reagent (MIR 2700, Mirus Bio LLC, Madison, WI) in 3 ml Optimem I media (Fisher Scientific Co LLC, Waltham, MA) were prepared by combining and vortexing for 2 seconds. The mixture was then incubated at RT for 30 minutes. The mixture was then mixed again gently and added dropwise to the plated HEK293T cells. On day three, the complete media was exchanged with fresh complete media. On day four, the supernatant was harvested from the cells and kept at 4°C. Fresh complete media was added to the cells. On day five, the supernatant was again harvested from the cells and combined with supernatant from day four. The supernatants were centrifuged at 13000 rpm to remove cell debris and transferred to a new tube. The supernatants were then filtered through a 0.45um/26mm syringe filter (Cole-Parmer). After filtering, supernatants were concentrated using Lenti-X^{™} Concentrator reagent (Clontech, 631232). 200 µl of Lenti-X^{™} Concentrator reagent was added per 800 µl supernatant and gently mixed. The mixture was then incubated on ice for 1hr, then centrifuged at 1500G for 45 minutes. The supernatant was removed and the pellet that contained the packaged concentrated virus used for further infections.

### Primary T cell transduction with lentivirus

Primary activated T cells were obtained from PBMCs as described in Example 1. 1.5 × 10⁷ primary activated T cells were infected with 1 batch of lentivirus made from 5 × 15 cm plates of packaged, concentrated virus. 1.5 × 10⁷ primary activated T cells were diluted in 15 ml complete media (Complete RPMI + 40 U/mL rhIL-2) supplemented with 5µg/ml Polybrene^{™} (Santa Cruz Biotechnology, Inc., sc-134220). The T cell suspension was used to resuspend the packaged concentrated virus pellet in a 50 ml Falcon tube. The suspension was centrifuged at 30°C at 1200g for 2 hours. Then the supernatant was gently remixed with the cell pellet and the suspension transferred to a non-tissue culture coated T25 flask. Flasks were then placed into a 37°C, 5% CO2 incubator. The next day, the transduced T cells were transferred to 50 mL conical tubes and centrifuged at 300 × g for approximately 7-10 minutes to pellet cells. The supernatant was discarded and the pellet was gently resuspended and the T cells pooled in an appropriate volume of complete RPMI and enumerated.

The enumerated T cells were resuspended at 1 × 10⁶ cells/mL in Complete RPMI + 40 U/mL rhIL-2 and plated into as many T-175 suspension flask as required (maximum volume per flask was 250 mL).

### CAR19-T cell nucleofection with NATNA/dCas9-VP64 targeting CCR2

Lentivirus transduced primary activated CAR19-T cells are nucleofected with NATNA/dCas9-VP64 targeting the CCR2 transcription activation region described in Example 3.

### FACS for CD3+CD4+CCR2+CAR19+ and CD3+CD8+CCR2+CAR19+ T cells

The nucleofected and lentivirus transduced primary activated CAR19-T cells are now positive for the following cell surface markers: CD3+, CD4+ or CD8+, CAR19+, and CCR2+. Cell surface expression of these markers is confirmed by FACS analysis as described in Example 4 using the appropriate primary and secondary antibodies for the chosen cell surface markers.

### Cytotoxicity assay using Raji CD19+ cells and CCR2+CAR19+ CAR-T cells

Cytotoxicity assays using lymphocytes and CAR-T cells have been described in the literature. Functional CAR-T cells are able to specifically kill CD19+ target cells. To test the cytotoxic potential of the CCR2+CAR19+CAR-T cells, a FACS based cytotoxicity assay is chosen. The method is adapted from Kochenderfer et al., J. Immunother. (2009) 32:689-702.

Raji cells are CD19+ and are used as target cells; K562 cells are CD19- and are used as control cells. CAR19-T cells and mock-transfected, activated T cells from the same donor are used as effector cells and control. Raji cells, K562 cells, CAR19-T cells and control T cells are grown in Complete RPMI + 40 U/mL rhIL-2 media at 37°C with 5% CO₂ at a cell density of between 1 × 10⁶ cells/ml and 3 × 10⁶ cells/ml. Target cells (Raji and K562 cells) are encoded with an appropriate cell dye such as CellTrace violet using the CellTrace Violet kit following the instructions in the manufacturer's protocol (ThermoFisher Scientific, Waltham, MA). Cells are enumerated using the Countess II FL Automated Cell Counter (ThermoFisher Scientific, Waltham, MA). 20.000 target cells (K562 and Raji) are plated per well in a 96 well tissue culture plate. Effector cells are added at several effector: target ratios such as such as 20:1, 10:1, 5:1, 3:1, 2:1 and 1:1, with a total reaction volume of 100 µL in Complete RPMI + 40 U/mL rhIL-2 media and incubated for up to 48 hours at 37°C with 5% CO₂. Cell viability is assessed by staining the cell mixture in each well with a cell viability dye such as a propidium iodide kit (BioLegend, San Diego, CA), following the instructions in the manufacturer's protocol. After 24-48 hours, the cell mixture is analyzed using FACS using an appropriate flow cytometer, such as the iQue Screener Plus^{™} (IntelliCyt, Albuquerque, NM). Target cells and effector cells are discriminated using CellTrace Violet staining. Then the fraction of live and dead cells within in each cell population is determined using expression of the viability dye.

Following the guidance of the present Specification and Examples, other altered lymphocytes can be used for targeted killing of other cell types by one of ordinary skill in the art.

**Table 8: Sequences referred to in the present Specification**

| **SEQ ID NO:** | **Name** | **Sequence** | **Locus** |
|---|---|---|---|
| | | | GRCh38.p7 |
| SEQ ID NO:1 | CCL2 | MKVSAALLCLLLIAATFIPQGLAQPDAINAPVTCCYNFTNRKISVQ RLASYRRITSSKCPKEAVIFKTIVAKEICADPKQKWVQDSMDHLDK QTQTPKT | NC_000017.11 (34255277..34 257203) |
| SEQ ID NO:2 | CCL3 | MQVSTAALAVLLCTMALCNQFSASLAADTPTACCFSYTSRQIPQNF IADYFETSSQCSKPGVIFLTKRSRQVCADPSEEWVQKYVSDLELSA | NC_000017.11 (36088256..36 090160, complement) |
| SEQ ID NO:3 | CCL4 | MKLCVTVLSLLMLVAAFCSPALSAPMGSDPPTACCFSYTARKLPRN FVVDYYETSSLCSQPAVVFQTKRSKQVCADPSESWVQEYVYDLELN | NC_000017.11 (36103827..36 105621) |
| SEQ ID NO:4 | CCL5 | MKVSAAALAVILIATALCAPASASPYSSDTTPCCFAYIARPLPRAH IKEYFYTSGKCSNPAVVFVTRKNRQVCANPEKKWVREYINSLEMS | NC_000017.11 (35871491..35 880373, complement) |
| SEQ ID NO:5 | CXCL8 | MTSKLAVALLAAFLISAALCEGAVLPRSAKELRCQCIKTYSKPFHP KFIKELRVIESGPHCANTEIIVKLSDGRELCLDPKENWVQRVVEKF LKRAENS | NC_000004.12 (73740506..73 743716) |
| SEQ ID NO:6 | CXCL10 | MNQTAILICCLIFLTLSGIQGVPLSRTVRCTCISISNQPVNPRSLE KLEIIPASQFCPRVEIIATMKKKGEKRCLNPESKAIKNLLKAVSKE RSKRSP | NC_000004.12 (76021116..76 023536, complement) |
| SEQ ID NO:7 | CXCL12 | MNAKVVVVLVLVLTALCLSDGKPVSLSYRCPCRFFESHVARANVKH LKILNTPNCALQIVARLKNNNRQVCIDPKLKWIQEYLEKALNKRFK M | NC_000010.11 (44292088..44 385097, complement) |
| SEQ ID NO:8 | TARC/C CL17 | MAPLKMLALVTLLLGASLQHIHAARGTNVGRECCLEYFKGAIPLRK LKTWYQTSEDCSRDAIVFVTVQGRAICSDPNNKRVKNAVKYLQSLE RS | NC_000016.10 (57396076..57 416063) |
| SEQ ID NO:9 | CCR1 | METPNTTEDYDTTTEFDYGDATPCQKVNERAFGAQLLPPLYSLVFV IGLVGNILVVLVLVQYKRLKNMTSIYLLNLAISDLLFLFTLPFWID YKLKDDWVFGDAMCKILSGFYYTGLYSEIFFIILLTIDRYLAIVHA VFALRARTVTFGVITSIIIWALAILASMPGLYFSKTQWEFTH HTCSLHFPHESLREWKLFQALKLNLFGLVLPLLVMIICYTGIIKIL LRRPNEKKSKAVRLIFVIMIIFFLFWTPYNLTILISVFQDFLFTHE CEQSRHLDLAVQVTEVIAYTHCCVNPVIYAFVGERFRKYLRQLFHR RVAVHLVKWLPFLSVDRLERVSSTSPSTGEHELSAGF | NC_ 000003.12 (46201709..46 208341, complement) |
| SEQ ID NO:10 | CCR2 | MLSTSRSRFIRNTNESGEEVTTFFDYDYGAPCHKFDVKQIGAQLLP PLYSLVFIFGFVGNMLVVLILINCKKLKCLTDIYLLNLAISDLLFL ITLPLWAHSAANEWVFGNAMCKLFTGLYHIGYFGGIFFIILLTIDR YLAIVHAVFALKARTVTFGVVTSVITWLVAVFASVPGIIFTK CQKEDSVYVCGPYFPRGWNNFHTIMRNILGLVLPLLIMVICYSGIL KTLLRCRNEKKRHRAVRVIFTIMIVYFLFWTPYNIVILLNTFQEFF GLSNCESTSQLDQATQVTETLGMTHCCINPIIYAFVGEKFRSLFHI ALGCRIAPLQKPVCGGPGVRPGKNVKVTTQGLLDGRGKGKSI GRAPEASLQDKEGA | NC_000003.12 (46353744..46 360940) |
| SEQ ID NO:11 | CCR3 | MTTSLDTVETFGTTSYYDDVGLLCEKADTRALMAQFVPPLYSLVF TVGLLGNVVVVMILIKYRRLRIMTNIYLLNLAISDLLFLVTLPFW IHYVRGHNWVFGHGMCKLLSGFYHTGLYSEIFFIILLTIDRYLAI VHAVFALRARTVTFGVITSIVTWGLAVLAALPEFIFYETEELFEE TLCSALYPEDTVYSWRHFHTLRMTIFCLVLPLLVMAICYTGIIKT LLRCPSKKKYKAIRLIFVIMAVFFIFWTPYNVAILLSSYQSILFG NDCERSKHLDLVMLVTEVIAYSHCCMNPVIYAFVGERFRKYLRHF FHRHLLMHLGRYIPFLPSEKLERTSSVSPSTAEPELSIVF | NC_000003.12 (46210699..46 266706) |
| SEQ ID NO:12 | CCR4 | MNPTDIADTTLDESIYSNYYLYESIPKPCTKEGIKAFGELFLPPL YSLVFVFGLLGNSVVVLVLFKYKRLRSMTDVYLLNLAISDLLFVF SLPFWGYYAADQWVFGLGLCKMISWMYLVGFYSGIFFVMLMSIDR YLAIVHAVFSLRARTLTYGVITSLATWSVAVFASLPGFLFSTCYT ERNHTYCKTKYSLNSTTWKVLSSLEINILGLVIPLGIMLFCYSMI IRTLQHCKNEKKNKAVKMIFAVVVLFLGFWTPYNIVLFLETLVEL EVLQDCTFERYLDYAIQATETLAFVHCCLNPIIYFFLGEKFRKYI LQLFKTCRGLFVLCQYCGLLQIYSADTPSSSYTQSTMDHDLHDAL | NC_000003.12 (32951555..32 955312) |
| SEQ ID NO:13 | CCR5 | | NC_000003.12 (46370142..46 376206) |
| SEQ ID NO:14 | CXCR1 | MSNITDPQMWDFDDLNFTGMPPADEDYSPCMLETETLNKYVVIIA YALVFLLSLLGNSLVMLVILYSRVGRSVTDVYLLNLALADLLFAL TLPIWAASKVNGWIFGTFLCKVVSLLKEVNFYSGILLLACISVDR YLAIVHATRTLTQKRHLVKFVCLGCWGLSMNLSLPFFLFRQAYHP NNSSPVCYEVLGNDTAKWRMVLRILPHTFGFIVPLFVMLFCYGFT LRTLFKAHMGQKHRAMRVIFAVVLIFLLCWLPYNLVLLADTLMRT QVIQESCERRNNIGRALDATEILGFLHSCLNPIIYAFIGQNFRHG FLKILAMHGLVSKEFLARHRVTSYTSSSVNVSSNL | NC_000002.12 (218162845..2 18166993, complement) |
| SEQ ID NO:15 | CXCR3 | MVLEVSDHQVLNDAEVAALLENFSSSYDYGENESDSCCTSPPCPQ DFSLNFDRAFLPALYSLLFLLGLLGNGAVAAVLLSRRTALSSTDT FLLHLAVADTLLVLTLPLWAVDAAVQWVFGSGLCKVAGALFNINF YAGALLLACISFDRYLNIVHATQLYRRGPPARVTLTCLAVWGLCL LFALPDFIFLSAHHDERLNATHCQYNFPQVGRTALRVLQLVAGFL LPLLVMAYCYAHILAVLLVSRGQRRLRAMRLVVVVVVAFALCWTP YHLVVLVDILMDLGALARNCGRESRVDVAKSVTSGLGYMHCCLNP LLYAFVGVKFRERMWMLLLRLGCPNQRGLQRQPSSSRRDSSWSET SEASYSGL | NC_000023.11 (71615913..71 618517, complement) |
| SEQ ID NO:16 | CXCR4 | MEGISIYTSDNYTEEMGSGDYDSMKEPCFREENANFNKIFLPTIY SIIFLTGIVGNGLVILVMGYQKKLRSMTDKYRLHLSVADLLFVIT LPFWAVDAVANWYFGNFLCKAVHVIYTVNLYSSVLILAFISLDRY LAIVHATNSQRPRKLLAEKVVYVGVWIPALLLTIPDFIFANVSEA DDRYICDRFYPNDLWVVVFQFQHIMVGLILPGIVILSCYCIIISK LSHSKGHQKRKALKTTVILILAFFACWLPYYIGISIDSFILLEII KQGCEFENTVHKWISITEALAFFHCCLNPILYAFLGAKFKTSAQH ALTSVSRGSSLKILSKGKRGGHSSVSTESESSSFHSS | NC_000002.12 (136114349..1 36118155, complement) |
| SEQ ID NO:17 | DARC | MGNCLHRAELSPSTENSSQLDFEDVWNSSYGVNDSFPDGDYGANL EAAAPCHSCNLLDDSALPFFILTSVLGILASSTVLFMLFRPLFRW QLCPGWPVLAQLAVGSALFSIVVPVLAPGLGSTRSSALCSLGYCV WYGSAFAQALLLGCHASLGHRLGAGQVPGLTLGLTVGIWGVAALL TLPVTLASGASGGLCTLIYSTELKALQATHTVACLAIFVLLPLGL FGAKGLKKALGMGPGPWMNILWAWFIFWWPHGVVLGLDFLVRSKL LLLSTCLAQQALDLLLNLAEALAILHCVATPLLLALFCHQATRTL LPSLPLPEGWSSHLDTLGSKS | NC_000001.11 (159204013..1 59206500) |
| SEQ ID NO:18 | VP64 | DALDDFDLDMLGSDALDDFDLDMLGSDALDDFDLDMLGSDALDDF DLDML | Synthetic |
| SEQ ID NO:19 | Spy dCas9 | DKKYSIGLAIGTNSVGWAVITDEYKVPSKKFKVLGNTDRHSIKKN LIGALLFDSGETAEATRLKRTARRRYTRRKNRICYLQEIFSNEMA KVDDSFFHRLEESFLVEEDKKHERHPIFGNIVDEVAYHEKYPTIY HLRKKLVDSTDKADLRLIYLALAHMIKFRGHFLIEGDLNPDNSDV DKLFIQLVQTYNQLFEENPINASGVDAKAILSARLSKSRRLENLI AQLPGEKKNGLFGNLIALSLGLTPNFKSNFDLAEDAKLQLSKDTY DDDLDNLLAQIGDQYADLFLAAKNLSDAILLSDILRVNTEITKAP LSASMIKRYDEHHQDLTLLKALVRQQLPEKYKEIFFDQSKNGYAG YIDGGASQEEFYKFIKPILEKMDGTEELLVKLNREDLLRKQRTFD NGSIPHQIHLGELHAILRRQEDFYPFLKDNREKIEKILTFRIPYY VGPLARGNSRFAWMTRKSEETITPWNFEEVVDKGASAQSFIERMT NFDKNLPNEKVLPKHSLLYEYFTVYNELTKVKYVTEGMRKPAFLS GEQKKAIVDLLFKTNRKVTVKQLKEDYFKKIECFDSVEISGVEDR FNASLGTYHDLLKIIKDKDFLDNEENEDILEDIVLTLTLFEDREM IEERLKTYAHLFDDKVMKQLKRRRYTGWGRLSRKLINGIRDKQSG KTILDFLKSDGFANRNFMQLIHDDSLTFKEDIQKAQVSGQGDSLH EHIANLAGSPAIKKGILQTVKVVDELVKVMGRHKPENIVIEMARE NQTTQKGQKNSRERMKRIEEGIKELGSQILKEHPVENTQLQNEKL YLYYLQNGRDMYVDQELDINRLSDYDVDAIVPQSFLKDDSIDNKV LTRSDKNRGKSDNVPSEEVVKKMKNYWRQLLNAKLITQRKFDNLT KAERGGLSELDKAGFIKRQLVETRQITKHVAQILDSRMNTKYDEN DKLIREVKVITLKSKLVSDFRKDFQFYKVREINNYHHAHDAYLNA VVGTALIKKYPKLESEFVYGDYKVYDVRKMIAKSEQEIGKATAKY FFYSNIMNFFKTEITLANGEIRKRPLIETNGETGEIVWDKGRDFA TVRKVLSMPQVNIVKKTEVQTGGFSKESILPKRNSDKLIARKKDW DPKKYGGFDSPTVAYSVLVVAKVEKGKSKKLKSVKELLGITIMER SSFEKNPIDFLEAKGYKEVKKDLIIKLPKYSLFELENGRKRMLAS AGELQKGNELALPSKYVNFLYLASHYEKLKGSPEDNEQKQLFVEQ HKHYLDEIIEQISEFSKRVILADANLDKVLSAYNKHRDKPIREQA ENIIHLFTLTNLGAPAAFKYFDTTIDRKRYTSTKEVLDATLIHQS ITGLYETRIDLSQLGGD | |
| SEQ ID NO:20 | 2x NLS | PKKKRKVDGSPKKKRKVDSG | Synthetic |
| SEQ ID NO:21 | CCR2 Target 1 | GGTATCATGGATTCAGAATCTGG | |
| SEQ ID NO:22 | CCR2 Target 2 | TGCTGGTTTCAGTGGTATCATGG | |
| SEQ ID NO:23 | CCR2 Target 3 | ATCATGTGTGCTGGTTTCAGTGG | |
| SEQ ID NO:24 | CCR2 Target 4 | TTTACTGCGATCATGTGTGCTGG | |
| SEQ ID NO:25 | CCR2 Target 5 | ATAG T GGAGGAAG TATAAT GAGG | |
| SEQ ID NO:26 | CCR2 Target 6 | AAGGGTATTGGTGATAGTGGAGG | |
| SEQ ID NO:27 | CCR2 Target 7 | ATAAAGGGTATTGGTGATAGTGG | |
| SEQ ID NO:28 | CCR2 Target 8 | CACCAATACCCTTTATTCTCTGG | |
| SEQ ID NO:29 | CCR2 Target 9 | TTCCAGAGAATAAAGGGTATTGG | |
| SEQ ID NO:30 | CCR2 Target 10 | TTCATGTTCCAGAGAATAAAGGG | |
| SEQ ID NO:31 | CCR2 Target 11 | TTTCATGTTCCAGAGAATAAAGG | |
| SEQ ID NO:32 | CCR2 Target 12 | TCATGCAAATTATCACTAGTAGG | |
| SEQ ID NO:33 | CCR2 Target 13 | ACTAGTAGGAGAGCAGAGAGTGG | |
| SEQ ID NO:34 | CCR2 Target 14 | GCAGAGAGTGGAAATGTTCCAGG | |
| SEQ ID NO:35 | CCR2 Target 15 | ATCTTGTGGGTCTTTATACCTGG | |
| SEQ ID NO:36 | CCR2 Target 16 | TCTGAGCTTCTTTATCTTGTGGG | |
| SEQ ID NO:37 | CCR2 Target 17 | CTCTGAGCTTCTTTATCTTGTGG | |
| SEQ ID NO:38 | CCR2 Target 18 | AGCTCAGAGTCGTTAGAAACAGG | |
| SEQ ID NO:39 | CCR2 Target 19 | AGAAACAGGAGCAGATGTACAGG | |
| SEQ ID NO:40 | CCR2 Target 20 | GAAACAGGAGCAGATGTACAGGG | |
| SEQ ID NO:41 | CCR2 Target 21 | GTTTGCCTGACTCACACTCAAGG | |
| SEQ ID NO:42 | CCR2 Target 22 | TGCAACCTTGAGTGTGAGTCAGG | |
| SEQ ID NO:43 | CCR2 Target 23 | TTTCAAAATTAATCCTATTCTGG | |
| SEQ ID NO:44 | CCR2 Target 24 | TGGGTTGAGGTCTCCAGAATAGG | |
| SEQ ID NO:45 | CCR2 Target 25 | GAACATTGTACATTGGGTTGAGG | |
| SEQ ID NO:46 | CCR2 Target 26 | AGTCAGGAACATTGTACATTGGG | |
| SEQ ID NO:47 | CCR2 Target 27 | CAGTCAGGAACATTGTACATTGG | |
| SEQ ID NO:48 | CCR2 Target 28 | CAATGTACAATGTTCCTGACTGG | |
| SEQ ID NO:49 | CCR2 Target 29 | ATAGTTCTTCTTTTCCAGTCAGG | |
| SEQ ID NO:50 | CCR2 Target 30 | CGGAGATACAGGGCAACTAATGG | |
| SEQ ID NO:51 | CCR2 Target 31 | AAAGTGAAGGCGGAGATACAGGG | |
| SEQ ID NO:52 | CCR2 Target 32 | GAAAGTGAAGGCGGAGATACAGG | |
| SEQ ID NO:53 | CCR2 Target 33 | TCTCCGCCTTCACTTTCTGCAGG | |
| SEQ ID NO:54 | CCR2 Target 34 | TTTCCTGCAGAAAGTGAAGGCGG | |
| SEQ ID NO:55 | CCR2 Target 35 | AAGTTTCCTGCAGAAAGTGAAGG | |
| SEQ ID NO:56 | CCR2 Target 36 | GAAACTTGGCATGCAGAAGTAGG | |
| SEQ ID NO:57 | CCR2 Target 37 | CAGATCTAGAGGTAGAAACTTGG | |
| SEQ ID NO:58 | CCR2 Target 38 | TTTCTACCTCTAGATCTGTTTGG | |
| SEQ ID NO:59 | CCR2 Target 39 | ACTGAACCAAACAGATCTAGAGG | |
| SEQ ID NO:60 | CCR2 Target 40 | GCTGAGAAGCCTGACATACCAGG | |
| SEQ ID NO:61 | CCR2 Guide target 1 | GGTATCATGGATTCAGAATC | |
| SEQ ID NO:62 | CCR2 Guide target 2 | TGCTGGTTTCAGTGGTATCA | |
| SEQ ID NO:63 | CCR2 Guide target 3 | ATCATGTGTGCTGGTTTCAG | |
| SEQ ID NO:64 | CCR2 Guide target 4 | TTTACTGCGATCATGTGTGC | |
| SEQ ID NO:65 | CCR2 Guide target 5 | ATAGTGGAGGAAGTATAATG | |
| SEQ ID NO:66 | CCR2 Guide target 6 | AAGGGTATTGGTGATAGTGG | |
| SEQ ID NO:67 | CCR2 Guide target 7 | ATAAAGGGTATTGGTGATAG | |
| SEQ ID NO:68 | CCR2 Guide target 8 | CACCAATACCCTTTATTCTC | |
| SEQ ID NO:69 | CCR2 Guide target 9 | TTCCAGAGAATAAAGGGTAT | |
| SEQ ID NO:70 | CCR2 Guide target 10 | TTCATGTTCCAGAGAATAAA | |
| SEQ ID NO:71 | CCR2 Guide target 11 | TTTCATGTTCCAGAGAATAA | |
| SEQ ID NO:72 | CCR2 Guide target 12 | TCATGCAAATTATCACTAGT | |
| SEQ ID NO:73 | CCR2 Guide target 13 | ACTAGTAGGAGAGCAGAGAG | |
| SEQ ID NO:74 | CCR2 Guide target 14 | GCAGAGAGTGGAAATGTTCC | |
| SEQ ID NO:75 | CCR2 Guide target 15 | ATCTTGTGGGTCTTTATACC | |
| SEQ ID NO:76 | CCR2 Guide target 16 | TCTGAGCTTCTTTATCTTGT | |
| SEQ ID NO:77 | CCR2 Guide target 17 | CTCTGAGCTTCTTTATCTTG | |
| SEQ ID NO:78 | CCR2 Guide target 18 | AGCTCAGAGTCGTTAGAAAC | |
| SEQ ID NO:79 | CCR2 Guide target 19 | AGAAACAGGAGCAGATGTAC | |
| SEQ ID NO:80 | CCR2 Guide target 20 | GAAACAGGAGCAGATGTACA | |
| SEQ ID NO:81 | CCR2 Guide target 21 | GTTTGCCTGACTCACACTCA | |
| SEQ ID NO:82 | CCR2 Guide target 22 | TGCAACCTTGAGTGTGAGTC | |
| SEQ ID NO:83 | CCR2 Guide target 23 | TTTCAAAATTAATCCTATTC | |
| SEQ ID NO:84 | CCR2 Guide target 24 | TGGGTTGAGGTCTCCAGAAT | |
| SEQ ID NO:85 | CCR2 Guide target 25 | GAACATTGTACATTGGGTTG | |
| SEQ ID NO:86 | CCR2 Guide target 26 | AGTCAGGAACATTGTACATT | |
| SEQ ID NO:87 | CCR2 Guide target 27 | CAGTCAGGAACATTGTACAT | |
| SEQ ID NO:88 | CCR2 Guide target 28 | CAATGTACAATGTTCCTGAC | |
| SEQ ID NO:89 | CCR2 Guide target 29 | ATAGTTCTTCTTTTCCAGTC | |
| SEQ ID NO:90 | CCR2 Guide target 30 | CGGAGATACAGGGCAACTAA | |
| SEQ ID NO:91 | CCR2 Guide target 31 | AAAG T GAAGGC GGAGATACA | |
| SEQ ID NO:92 | CCR2 Guide target 32 | GAAAGTGAAGGCGGAGATAC | |
| SEQ ID NO:93 | CCR2 Guide target 33 | TCTCCGCCTTCACTTTCTGC | |
| SEQ ID NO:94 | CCR2 Guide target 34 | TTTCCTGCAGAAAGTGAAGG | |
| SEQ ID NO:95 | CCR2 Guide target 35 | AAGTTTCCTGCAGAAAGTGA | |
| SEQ ID NO:96 | CCR2 Guide target 36 | GAAACTTGGCATGCAGAAGT | |
| SEQ ID NO:97 | CCR2 Guide target 37 | CAGATCTAGAGGTAGAAACT | |
| SEQ ID NO:98 | CCR2 Guide target 38 | TTTCTACCTCTAGATCTGTT | |
| SEQ ID NO: 99 | CCR2 Guide target 39 | ACTGAACCAAACAGATCTAG | |
| SEQ ID NO: 100 | CCR2 Guide target 40 | GCTGAGAAGCCTGACATACC | |
| SEQ ID NO: 101 | CCR2 Guide target 1RNA | GGUAUCAUGGAUUCAGAAUCGUUUUAGAGCUAUGCU | |
| SEQ ID NO: 102 | CCR2 Guide target 2RNA | UGCUGGUUUCAGUGGUAUCAGUUUUAGAGCUAUGCU | |
| SEQ ID NO: 103 | CCR2 Guide target 3RNA | AUCAUGUGUGCUGGUUUCAGGUUUUAGAGCUAUGCU | |
| SEQ ID NO: 104 | CCR2 Guide target 4RNA | UUUACUGCGAUCAUGUGUGCGUUUUAGAGCUAUGCU | |
| SEQ ID NO: 105 | CCR2 Guide target 5RNA | AUAGUGGAGGAAGUAUAAUGGUUUUAGAGCUAUGCU | |
| SEQ ID NO: 106 | CCR2 Guide target 6RNA | AAGGGUAUUGGUGAUAGUGGGUUUUAGAGCUAUGCU | |
| SEQ ID NO: 107 | CCR2 Guide target 7RNA | AUAAAGGGUAUUGGUGAUAGGUUUUAGAGCUAUGCU | |
| SEQ ID NO: 108 | CCR2 Guide target 8RNA | CACCAAUACCCUUUAUUCUCGUUUUAGAGCUAUGCU | |
| SEQ ID NO: 109 | CCR2 Guide target 9RNA | UUCCAGAGAAUAAAGGGUAUGUUUUAGAGCUAUGCU | |
| SEQ ID NO: 110 | CCR2 Guide target 10RNA | UUCAUGUUCCAGAGAAUAAAGUUUUAGAGCUAUGCU | |
| SEQ ID NO: 111 | CCR2 Guide target 11RNA | UUUCAUGUUCCAGAGAAUAAGUUUUAGAGCUAUGCU | |
| SEQ ID NO: 112 | CCR2 Guide target 12RNA | UCAUGCAAAUUAUCACUAGUGUUUUAGAGCUAUGCU | |
| SEQ ID NO: 113 | CCR2 Guide target 13RNA | ACUAGUAGGAGAGCAGAGAGGUUUUAGAGCUAUGCU | |
| SEQ ID NO: 114 | CCR2 Guide target 14RNA | GCAGAGAGUGGAAAUGUUCCGUUUUAGAGCUAUGCU | |
| SEQ ID NO: 115 | CCR2 Guide target 15RNA | AUCUUGUGGGUCUUUAUACCGUUUUAGAGCUAUGCU | |
| SEQ ID NO: 116 | CCR2 Guide target 16RNA | UCUGAGCUUCUUUAUCUUGUGUUUUAGAGCUAUGCU | |
| SEQ ID NO: 117 | CCR2 Guide target 17RNA | CUCUGAGCUUCUUUAUCUUGGUUUUAGAGCUAUGCU | |
| SEQ ID NO: 118 | CCR2 Guide target 18RNA | AGCUCAGAGUCGUUAGAAACGUUUUAGAGCUAUGCU | |
| SEQ ID NO: 119 | CCR2 Guide target 19RNA | AGAAACAGGAGCAGAUGUACGUUUUAGAGCUAUGCU | |
| SEQ ID NO: 120 | CCR2 Guide target 2 0 RNA | GAAACAGGAGCAGAUGUACAGUUUUAGAGCUAUGCU | |
| SEQ ID NO: 121 | CCR2 Guide target 21RNA | GUUUGCCUGACUCACACUCAGUUUUAGAGCUAUGCU | |
| SEQ ID NO: 122 | CCR2 Guide target 22RNA | UGCAACCUUGAGUGUGAGUCGUUUUAGAGCUAUGCU | |
| SEQ ID NO: 123 | CCR2 Guide target 23RNA | UUUCAAAAUUAAUCCUAUUCGUUUUAGAGCUAUGCU | |
| SEQ ID NO: 124 | CCR2 Guide target 24RNA | UGGGUUGAGGUCUCCAGAAUGUUUUAGAGCUAUGCU | |
| SEQ ID NO: 125 | CCR2 Guide target 25RNA | GAACAUUGUACAUUGGGUUGGUUUUAGAGCUAUGCU | |
| SEQ ID NO: 126 | CCR2 Guide target 26RNA | AGUCAGGAACAUUGUACAUUGUUUUAGAGCUAUGCU | |
| SEQ ID NO: 127 | CCR2 Guide target 27RNA | CAGUCAGGAACAUUGUACAUGUUUUAGAGCUAUGCU | |
| SEQ ID NO: 128 | CCR2 Guide target 28RNA | CAAUGUACAAUGUUCCUGACGUUUUAGAGCUAUGCU | |
| SEQ ID NO: 129 | CCR2 Guide target 29RNA | AUAGUUCUUCUUUUCCAGUCGUUUUAGAGCUAUGCU | |
| SEQ ID NO: 130 | CCR2 Guide target 30RNA | CGGAGAUACAGGGCAACUAAGUUUUAGAGCUAUGCU | |
| SEQ ID NO: 131 | CCR2 Guide target 31RNA | AAAGUGAAGGCGGAGAUACAGUUUUAGAGCUAUGCU | |
| SEQ ID NO: 132 | CCR2 Guide target 32RNA | GAAAGUGAAGGCGGAGAUACGUUUUAGAGCUAUGCU | |
| SEQ ID NO: 133 | CCR2 Guide target 33RNA | UCUCCGCCUUCACUUUCUGCGUUUUAGAGCUAUGCU | |
| SEQ ID NO: 134 | CCR2 Guide target 34RNA | UUUCCUGCAGAAAGUGAAGGGUUUUAGAGCUAUGCU | |
| SEQ ID NO: 135 | CCR2 Guide target 35RNA | AAGUUUCCUGCAGAAAGUGAGUUUUAGAGCUAUGCU | |
| SEQ ID NO: 136 | CCR2 Guide target 36RNA | GAAACUUGGCAUGCAGAAGUGUUUUAGAGCUAUGCU | |
| SEQ ID NO: 137 | CCR2 Guide target 37RNA | CAGAUCUAGAGGUAGAAACUGUUUUAGAGCUAUGCU | |
| SEQ ID NO: 138 | CCR2 Guide target 38RNA | UUUCUACCUCUAGAUCUGUUGUUUUAGAGCUAUGCU | |
| SEQ ID NO: 139 | CCR2 Guide target 39RNA | ACUGAACCAAACAGAUCUAGGUUUUAGAGCUAUGCU | |
| SEQ ID NO: 140 | CCR2 Guide target 40RNA | GCUGAGAAGCCUGACAUACCGUUUUAGAGCUAUGCU | |
| SEQ ID NO: 141 | tracr RNA | AGCAUAGCAAGUUAAAAUAAGGCUAGUCCGUUAUCAACUUGAAAA AGUGGCACCGAGUCGGUGCUU | |

## Claims

1. A modified human T cell comprising at least one ectopically expressed endogenous chemokine receptor not expressed in an unmodified human T cell,
wherein the modified human T cell is produced by a method comprising introducing into a human T cell a polynucleotide encoding a chimeric antigen receptor (CAR); and an artificial transcription factor (ATF) complex comprising a catalytically inactive CRISPR-associated protein (dCas), a nucleic acid-targeting nucleic acid (NATNA), and an effector domain; whereby the ATF complex binds to a nucleic acid proximal to a transcriptional start site (TSS) of an endogenous chemokine receptor gene,
wherein the at least one ectopically expressed endogenous chemokine receptor is selected from the group consisting of CCR1, CCR2, CCR3, CCR4, CCR5, CXCR4 and DARC.

2. The modified human T cell of claim 1, wherein the CAR is selected from the group consisting of anti-CD19 CAR, anti-CD20 CAR, anti-CD22 CAR, anti-CD30 CAR, anti-CD33 CAR, anti-CD138 CAR, anti-CD171 CAR, anti-CEA CAR, anti-CD123 CAR, IL13 CAR, anti-epidermal growth factor receptor CAR, anti-EGFRvIII CAR, anti-ErbB CAR, anti-FAP CAR, anti-GD2 CAR, anti-glypican 3 CAR, anti-Her2 CAR, anti-mesothelin CAR, NKG2D CAR, anti-PD1 CAR, anti-MUC1 CAR, anti-VEGF2 CAR, and anti-ROR1 CAR.

3. A composition comprising:
the modified human T cell of claim 1; and
a pharmaceutically acceptable excipient.

4. The composition of claim 3 for use in the treatment of cancer comprising administering a therapeutically effective amount of said composition to a human subject in need thereof.

5. The composition of claim 3 for the use of claim 4, wherein the cancer is selected from the group consisting of prostate cancer, ovarian cancer, cervical cancer, colorectal cancer, intestinal cancer, testicular cancer, skin cancer, lung cancer, thyroid cancer, bone cancer, breast cancer, bladder cancer, uterine cancer, vaginal cancer, pancreatic cancer, liver cancer, kidney cancer, brain cancer, spinal cord cancer, oral cancer, parotid tumor, blood cancer, lymphomas, solid tumors, and liquid tumors.

6. The composition of claim 3 for the use of claim 4, wherein the composition is administered intravenously.

7. The modified human T cells of claim 1, wherein the method further comprises selecting the modified human T cells ectopically expressing the selected endogenous chemokine receptor gene on the modified human T cell surface.

8. The modified human T cells of claim 1, wherein the effector domain comprises VP16 or VP64.

9. The modified human T cells of claim 1, wherein the effector domain comprises MS2-binding RNA.

10. The modified human T cells of claim 1, wherein the selected endogenous chemokine receptor gene encodes CCR2.

11. The modified human T cells of claim 1, wherein the selected endogenous chemokine receptor gene encodes CXCR1.

12. The modified human T cells of claim 1, wherein the CAR is an anti-CD19 CAR.

## Patentansprüche

1. Eine modifizierte humane T-Zelle, umfassend mindestens einen ektopisch exprimierten endogenen Chemokinrezeptor, der in einer nicht-modifizierten T-Zelle nicht exprimiert wird,
wobei die modifizierte humane T-Zelle durch ein Verfahren hergestellt wird, das das Einführen eines Polynukleotids, das einen chimären Antigenrezeptor (CAR) kodiert; und eines artifiziellen Transkriptionsfaktor- (ATF) Komplexes, umfassend ein katalytisch inaktives CRISPR-assoziiertes Protein (dCas), eine Nukleinsäure, die eine Nukleinsäure ansteuert (NATNA) und eine Effektordomäne; in eine humane T-Zelle umfasst; wobei der ATF-Komplex an eine Nukleinsäure proximal der Transkriptionsstartstelle (TSS) eines endogenen Chemokinrezeptorgens bindet,
wobei der mindestens eine ektopisch exprimierte endogene Chemokinrezeptor ausgewählt ist aus der Gruppe bestehend aus CCR1, CCR2, CCR3, CCR4, CCR5, CXCR4 und DARC.

2. Die modifizierte humane T-Zelle nach Anspruch 1, wobei der CAR ausgewählt ist aus der Gruppe bestehend aus anti-CD19 CAR, anti-CD20 CAR, anti-CD22 CAR, anti-CD30 CAR, anti-CD33 CAR, anti-CD138 CAR, anti-CD171 CAR, anti-CEA CAR, anti-CD123 CAR, IL13 CAR, anti-epidermaler Wachstumsfaktorrezeptor CAR, anti-EGFRvIII CAR, anti-ErbB CAR, anti-FAP CAR, anti-GD2 CAR, anti-Glypican 3 CAR, anti-Her2 CAR, anti-Mesothelin CAR, NKG2D CAR, anti-PD1 CAR, anti-MUC1 CAR, anti-VEGF2 CAR, und anti-ROR1 CAR.

3. Eine Zusammensetzung umfassend:
die modifizierte humane T-Zelle nach Anspruch 1; und
einen pharmazeutisch annehmbaren Auszugsstoff.

4. Die Zusammensetzung nach Anspruch 3 zur Verwendung bei der Behandlung von Krebs, umfassend das Verabreichen einer therapeutisch effektiven Menge jener Zusammensetzung an ein humanes Subjekt, das daran Bedarf hat.

5. Die Zusammensetzung nach Anspruch 3 zur Verwendung nach Anspruch 4, wobei der Krebs ausgewählt ist aus der Gruppe bestehend aus Prostatakrebs, Eierstockkrebs, Gebärmutterhalskrebs, Dickdarmkrebs, Darmkrebs, Hodenkrebs, Hautkrebs, Lungenkrebs, Schilddrüsenkrebs, Knochenkrebs, Brustkrebs, Blasenkrebs, Gebärmutterkrebs, Vaginalkrebs, Bauchspeicheldrüsenkrebs, Leberkrebs, Nierenkrebs, Gehirnkrebs, Rückenmarkskrebs, Mundhöhlenkrebs, Ohrspeicheldrüsentumor, Blutkrebs, Lymphome, feste Tumore und liquide Tumore.

6. Die Zusammensetzung nach Anspruch 3 zur Verwendung nach Anspruch 4, wobei die Zusammensetzung intravenös verabreicht wird.

7. Die modifizierten humanen T-Zellen nach Anspruch 1, wobei das Verfahren ferner das Auswählen der modifizierten humanen T-Zellen, die das ausgewählte endogene Chemokinrezeptorgen auf der modifizierten humanen T-Zell-Oberfläche ektopisch exprimieren, umfasst.

8. Die modifizierten humanen T-Zellen nach Anspruch 1, wobei die Effektordomäne VP16 oder VP64 umfasst.

9. Die modifizierten humanen T-Zellen nach Anspruch 1, wobei die Effektordomäne MS2-bindende RNA umfasst.

10. Die modifizierten humanen T-Zellen nach Anspruch 1, wobei das ausgewählte endogene Chemokinrezeptorgen CCR2 kodiert.

11. Die modifizierten humanen T-Zellen nach Anspruch 1, wobei das ausgewählte endogene Chemokinrezeptorgen CXCR1 kodiert.

12. Die modifizierten humanen T-Zellen nach Anspruch 1, wobei der CAR ein anti-CD19 CAR ist.

## Revendications

1. Cellule T humaine modifiée comprenant au moins un récepteur de chimiokine endogène exprimé de manière ectopique, non exprimé dans une cellule T humaine non modifiée,
dans lequel la cellule T humaine modifiée est produit par un procédé comprenant l'introduction dans une cellule T humaine d'un polynucléotide codant pour un récepteur antigénique chimérique (CAR) ; et un complexe de facteur de transcription artificiel (ATF) comprenant une protéine associée à CRISPR (dCas) catalytiquement inactive, un acide nucléique de ciblage d'acide nucléique (NATNA) et un domaine effecteur; moyennant quoi le complexe ATF se lie à un acide nucléique à proximité d'un site d'initiation de transcription (TSS) d'un gène de récepteur de chimiokine endogène,
dans lequel le au moins un récepteur de chimiokine endogènes exprimé de manière ectopique est sélectionné dans le groupe consistant en CCR1, CCR2, CCR3, CCR4, CCR5, CXCR4 et DARC.

2. Cellule T humaine modifiée selon la revendication 1, dans lequel le CAR est sélectionné dans le groupe consistant en CAR anti-CD19, CAR anti-CD20, CAR anti-CD22, CAR anti-CD30, CAR anti-CD33, CAR anti-CD138, CAR anti-CD171, CAR anti-CEA, CAR anti-CD123, CAR IL13, CAR anti-récepteur du facteur de croissance épidermique, CAR anti-EGFRvIII, CAR anti-ErbB, CAR anti-FAP, CAR anti-GD2, CAR anti-glypicane 3, CAR anti-Her2, CAR anti-mésothéline, CAR NKG2D, CAR anti-PD1, CAR anti-MUC1, CAR anti-VEGF2, et CAR anti-ROR1.

3. Composition comprenant :
la cellule T humaine modifiée selon la revendication 1 ; et
un excipient pharmaceutiquement acceptable.

4. Composition selon la revendication 3 pour une utilisation dans le traitement d'un cancer, comprenant l'administration d'une quantité thérapeutiquement efficace de ladite composition à un sujet humain en ayant besoin.

5. Composition selon la revendication 3 pour une utilisation selon la revendication 4, dans laquelle le cancer est sélectionné dans le groupe consistant en un cancer de la prostate, un cancer des ovaires, un cancer du col de l'utérus, un cancer colorectal, un cancer de l'intestin, un cancer des testicules, un cancer de la peau, un cancer du poumon, un cancer de la thyroïde, un cancer des os, un cancer du sein, un cancer de la vessie, un cancer de l'utérus, un cancer du vagin, un cancer du pancréas, un cancer du foie, un cancer du rein, un cancer du cerveau, un cancer de la moelle épinière, un cancer buccal, une tumeur parotide, un cancer du sang, les lymphomes, les tumeurs solides, et les tumeurs liquides.

6. Composition selon la revendication 3 pour une utilisation selon la revendication 4, dans laquelle la composition est administrée par voie intraveineuse.

7. Cellules T humaines modifiées selon la revendication 1, dans lesquels le procédé comprend en outre la sélection des cellules T humaines modifiées exprimant de manière ectopique le gène du récepteur de chimiokine endogène sélectionné sur la surface des cellules T humaines modifiées.

8. Cellules T humaines modifiées selon la revendication 1, dans lesquels le domaine effecteur comprend VP16 ou VP64.

9. Cellules T humaines modifiées selon la revendication 1, dans lesquels le domaine effecteur comprend un ARN se liant à MS2.

10. Cellules T humaines modifiées selon la revendication 1, dans lesquels le gène du récepteur de chimiokine endogène sélectionné code pour CCR2.

11. Cellules T humaines modifiées selon la revendication 1, dans lesquels le gène du récepteur de chimiokine endogène sélectionné code pour CXCR1.

12. Cellules T humaines modifiées selon la revendication 1, dans lesquels le CAR est un CAR anti-CD19.
